# EUROPEAN PATENT APPLICATION

(11) **EP 4 198 055 A1**
(43) Date of publication of application: **21.06.2023**
(21) Application number: 21855588.6
(22) Date of filing: 12.08.2021
(51) Int. Cl.: C07K 16/24, C07K 14/715, C07K 14/54, A61P 17/02, A61P 35/00, A61P 43/00

(54) **ANTIBODY OF IL-11 AND USE THEREOF**

(30) Priority: 13.08.2020 CN 202010814358
(71) Applicant: Sunshine Lake Pharma Co., Ltd., Dongguan, Guangdong 523000 (CN)
(72) Inventor: ZHOU, Jiatao, Dongguan, Guangdong 523871 (CN); CHEN, Cangsha, Dongguan, Guangdong 523871 (CN); WANG, Shujuan, Dongguan, Guangdong 523871 (CN); YAO, Jie, Dongguan, Guangdong 523871 (CN); CHEN, Xiaofeng, Dongguan, Guangdong 523871 (CN); LI, Wenjia, Dongguan, Guangdong 523871 (CN)
(74) Representative: Hoffmann Eitle
(86) International application number: PCT/CN2021/112181
(87) International publication number: WO 2022/033538

(57) **Abstract**

Provided is an antibody or an antigen-binding fragment which can specifically recognize IL-11 thereof. The antibody contains CDR sequence(s) selected from at least one of the following or an amino acid sequence having at least 95% identity thereto: heavy chain variable region CDR sequences: SEQ ID NOs: 1-33 and light chain variable region CDR sequences: SEQ IN NOs: 34-66. The antibody can specifically target and bind to IL-11, so as to block binding of IL-11 and an IL-11 receptor.

## Description

### FIELD OF THE INVENTION

The present invention relates to the field of biotechnology, in particular, the present invention relates to an IL-11 antibody and use thereof, more particularly, the present invention relates to an antibody or an antigen-binding fragment thereof that can specifically recognize IL-11, a nucleic acid molecule, an expression vector, a recombinant cell, a pharmaceutical composition, pharmaceutical use, and a kit for detecting IL-11.

### BACKGROUND ART

Fibrosis is an important process and a critical part of wound healing. Excessive fibrosis is common in many rare and common diseases and is important in disease pathogenesis. Diseases characterized by excessive fibrosis include, but are not limited to: systemic sclerosis, scleroderma, hypertrophic cardiomyopathy, dilated cardiomyopathy (DCM), atrial fibrillation, ventricular fibrillation, myocarditis, cirrhosis, kidney disease, eye disease, asthma, cystic fibrosis, arthritis and idiopathic pulmonary fibrosis. Despite the high impact on human health, there is still an unmet medical need for treatments and diagnotics for fibrosis.

The true physiological role of interleukin 11 (IL-11) is unclear. IL-11 is most closely related to hematopoietic cell activation and thrombopoiesis, but has also been found to have pro- and anti-inflammatory, pro-angiogenic effects, and is important for neoplasia. TGFβ1 or tissue damage is known to induce the expression of IL-11. From the published literature, the role of IL-11 in fibrosis is unclear. IL-11 is thought to be important for pulmonary fibrosis and inflammation, and its expression levels correlate with collagen levels in the skin and respiratory system.

However, most studies suggest that IL-11 is anti-fibrotic in the heart and kidney, and anti-inflammatory in several tissues and chronic inflammatory diseases. In general, the molecular mode of action of IL-11 is thought to regulate the mRNA level of RNA expression through STAT3-mediated transcription.

Studies have demonstrated the administration of agents capable of inhibiting the action of interleukin 11 (IL-11) can treat, prevent or alleviate fibrosis in subjects in need of treatment. The agents capable of inhibiting the action of IL-11 can prevent or reduce the binding of IL-11 to the IL-11 receptor.

Currently the only drugs on the market for pulmonary fibrosis are pirfenidone and nintedanib, but these two drugs have poor efficacy, and drugs with a new mechanism are needed to solve this dilemma.

The development of agents with the effect of inhibiting IL-11 has become the direction of the development of drugs for the treatment and prevention of pulmonary fibrotic diseases.

### SUMMARY

The present invention aims to solve one of the technical problems in the related art at least to a certain extent. To this end, the present invention develops an Anti-IL-11 antibody with high neutralizing activity, which is expected to be used for the treatment or prevention of fibrosis-related diseases.

In the first aspect of the present invention, the present invention provides an antibody or an antigen-binding fragment thereof that can specifically recognize IL-11. According to the embodiments of the present invention, the antibody comprises CDRs selected from at least one of the following or an amino acid sequence having at least 95% identity thereto: a heavy chain variable region comprising CDRs having the amino acid sequence of SEQ ID NO: 1-33, a light chain variable region comprising CDRs having the amino acid sequence of SEQ IN NO: 34-66.
GYTFTTNG (SEQ ID NO:1).
INTNTGEP (SEQ ID NO:2).
AREGAFGLDY (SEQ ID NO:3).
DYTFT`NYW (SEQ ID NO:4).
IYPGGGYT (SEQ ID NO:5).
ARVRSGNDALDF (SEQ ID NO:6).
GYTFTNYG (SEQ ID NO:7).
INTNTGEP (SEQ ID NO:8).
SREGIYGMDS (SEQ ID NO:9).
GFSLTSYG (SEQ ID NO:10).
IWAGGRT (SEQ ID NO:11).
AREGGYDYDGDLWT (SEQ ID NO:12).
GFSLTSYG (SEQ ID NO: 13).
IWSGGIT (SEQ ID NO: 14).
ARNFDGYYYSIDY (SEQ ID NO: 15).
GFSLTNYG (SEQ ID NO:16).
IWRGGST (SEQ ID NO: 17).
AKNLYGPAAMDY (SEQ ID NO: 18).
GDTFTNYA (SEQ ID NO: 19).
IDTYTGDP (SEQ ID NO:20).
AGDTWFAY (SEQ ID NO:21).
GFSLSSSGMS (SEQ ID NO:22).
IWWSDDK (SEQ ID NO:23).
AREGSLGYGLDY (SEQ ID NO:24).
GFDFRRYW (SEQ ID NO:25).
INPDSSMI (SEQ ID NO:26).
ATYGHHATDS (SEQ ID NO:27).
GYTFTSYW (SEQ ID NO:28).
IYPGSDST (SEQ ID NO:29).
ALDSSGYGFAY (SEQ ID NO:30).
DYEFPSHD (SEQ ID NO:31).
INSDGGNT (SEQ ID NO:32).
ARPRPTIGTTATGSSM (SEQ ID NO:33).
QSVSND (SEQ ID NO:34).
YGS (SEQ ID NO:35).
QQDFFSPWT (SEQ ID NO:36).
ESVDEYGISF (SEQ ID NO:37).
SAS (SEQ ID NO:38).
QQSKEVPWT (SEQ ID NO:39).
QSVSND (SEQ ID NO:40).
YAS (SEQ ID NO:41).
QQDYYSPWT (SEQ ID NO:42).
QSLVHSNGNTY (SEQ ID NO:43).
KVS (SEQ ID NO:44).
SQSTHVPPD (SEQ ID NO:45).
QDISNS (SEQ ID NO:46).
YTS (SEQ ID NO:47).
QQGNTLPLT (SEQ ID NO:48).
QNIYVW (SEQ ID NO:49).
EAS (SEQ ID NO:50).
QQGQSYPYT (SEQ ID NO:51).
QSLLYSRNQKNR (SEQ ID NO:52).
WAS (SEQ ID NO:53).
QQYYSYPRT (SEQ ID NO:54).
QSIVHTDGNTY (SEQ ID NO:55).
KVS (SEQ ID NO:56).
FQGSHVPWT (SEQ ID NO:57).
ESVDSYGNSF (SEQ ID NO:58).
RAS (SEQ ID NO:59).
QQSNEDPFT (SEQ ID NO:60).
QSVSND (SEQ ID NO:61).
YAS (SEQ ID NO:62).
QQDYSSPFT (SEQ ID NO:63).
QSVSTSTYNY (SEQ ID NO:64).
YAS (SEQ ID NO:65).
QHSWEIPFT (SEQ ID NO:66).

The above-mentioned antibody according to the embodiments of the present invention can specifically target and bind to IL-11, and block the binding of IL-11 and an IL-11 receptor.

In another aspect, the present invention provides an antibody or an antigen-binding fragment thereof that can specifically recognize IL-11. According to the embodiments of the present invention, the antibody comprises CDRs selected from at least one of the following or an amino acid sequence having at least 95% identity thereto: a heavy chain variable region comprising CDRs having the amino acid sequence of SEQ ID NO: 1-33, a light chain variable region comprising CDRs having the amino acid sequence of SEQ IN NO: 34-66, the antibody or antigen-binding fragment thereof has humanization modifications. It should be noted that the "humanization modification" mentioned in this application refers to amino acid changes that can reduce the immunogenicity of the antibody or antigen-binding fragment thereof, including the mutation, insertion, deletion of amino acid, duplication of chemical group, and so on. The immunogenicity of the above-mentioned humanized antibody according to the embodiments of the present invention is reduced, and the antibody can specifically target and bind to IL-11, and block the binding of IL-11 and IL-11 receptor.

According to the embodiments of the present invention, the above-mentioned antibody or antigen-binding fragment may further include at least one of the following additional technical features:
According to the embodiments of the present invention, the antibody comprises:
a heavy chain variable region comprising CDR1, CDR2, CDR3 as shown in SEQ ID NO: 1, 2 and 3 or an amino acid sequence having at least 95% identity to SEQ ID NO: 1, 2 and 3, respectively; or
a heavy chain variable region comprising CDR1, CDR2, CDR3 as shown in SEQ ID NO: 4, 5 and 6 or an amino acid sequence having at least 95% identity to SEQ ID NO: 4, 5 and 6, respectively; or
a heavy chain variable region comprising CDR1, CDR2, CDR3 as shown in SEQ ID NO: 7, 8 and 9 or an amino acid sequence having at least 95% identity to SEQ ID NO: 7, 8 and 9, respectively; or
a heavy chain variable region comprising CDR1, CDR2, CDR3 as shown in SEQ ID NO: 10, 11 and 12 or an amino acid sequence having at least 95% identity to SEQ ID NO: 10, 11 and 12, respectively; or
a heavy chain variable region comprising CDR1, CDR2, CDR3 as shown in SEQ ID NO: 13, 14 and 15 or an amino acid sequence having at least 95% identity to SEQ ID NO: 13, 14 and 15, respectively; or
a heavy chain variable region comprising CDR1, CDR2, CDR3 as shown in SEQ ID NO: 16, 17 and 18 or an amino acid sequence having at least 95% identity to SEQ ID NO: 16, 17 and 18, respectively; or
a heavy chain variable region comprising CDR1, CDR2, CDR3 as shown in SEQ ID NO: 19, 20 and 21 or an amino acid sequence having at least 95% identity to SEQ ID NO: 19, 20 and 21, respectively; or
a heavy chain variable region comprising CDR1, CDR2, CDR3 as shown in SEQ ID NO: 22, 23 and 24 or an amino acid sequence having at least 95% identity to SEQ ID NO: 22, 23 and 24, respectively; or
a heavy chain variable region comprising CDR1, CDR2, CDR3 as shown in SEQ ID NO: 25, 26 and 27 or an amino acid sequence having at least 95% identity to SEQ ID NO: 25, 26 and 27, respectively; or
a heavy chain variable region comprising CDR1, CDR2, CDR3 as shown in SEQ ID NO: 28, 29 and 30 or an amino acid sequence having at least 95% identity to SEQ ID NO: 28, 29 and 30, respectively; or
a heavy chain variable region comprising CDR1, CDR2, CDR3 as shown in SEQ ID NO: 31, 32 and 33 or an amino acid sequence having at least 95% identity to SEQ ID NO: 31, 32 and 33.

According to the embodiments of the present invention, the antibody comprises:
a light chain variable region comprising CDR1, CDR2, CDR3 as shown in SEQ ID NO: 34, 35 and 36 or an amino acid sequence having at least 95% identity to SEQ ID NO: 34, 35 and 36, respectively; or
a light chain variable region comprising CDR1, CDR2, CDR3 as shown in SEQ ID NO: 37, 38 and 39 or an amino acid sequence having at least 95% identity to SEQ ID NO: 37, 38 and 39, respectively; or
a light chain variable region comprising CDR1, CDR2, CDR3 as shown in SEQ ID NO: 40, 41 and 42 or an amino acid sequence having at least 95% identity to SEQ ID NO: 40, 41 and 42, respectively; or
a light chain variable region comprising CDR1, CDR2, CDR3 as shown in SEQ ID NO: 43, 44 and 45 or an amino acid sequence having at least 95% identity to SEQ ID NO: 43, 44 and 45, respectively; or
a light chain variable region comprising CDR1, CDR2, CDR3 as shown in SEQ ID NO: 46, 47 and 48 or an amino acid sequence having at least 95% identity to SEQ ID NO: 46, 47 and 48, respectively; or
a light chain variable region comprising CDR1, CDR2, CDR3 as shown in SEQ ID NO: 49, 50 and 51 or an amino acid sequence having at least 95% identity to SEQ ID NO: 49, 50 and 51, respectively; or
a light chain variable region comprising CDR1, CDR2, CDR3 as shown in SEQ ID NO: 52, 53 and 54 or an amino acid sequence having at least 95% identity to SEQ ID NO: 52, 53 and 54, respectively; or
a light chain variable region comprising CDR1, CDR2, CDR3 as shown in SEQ ID NO: 55, 56 and 57 or an amino acid sequence having at least 95% identity to SEQ ID NO: 55, 56 and 57, respectively; or
a light chain variable region comprising CDR1, CDR2, CDR3 as shown in SEQ ID NO: 58, 59 and 60 or an amino acid sequence having at least 95% identity to SEQ ID NO: 58, 59 and 60, respectively; or
a light chain variable region comprising CDR1, CDR2, CDR3 as shown in SEQ ID NO: 61, 62 and 63 or an amino acid sequence having at least 95% identity to SEQ ID NO: 61, 62 and 63, respectively; or
a light chain variable region comprising CDR1, CDR2, CDR3 as shown in SEQ ID NO: 64, 65 and 66 or an amino acid sequence having at least 95% identity to SEQ ID NO: 64, 65 and 66, respectively.

According to the embodiments of the present invention, the above-mentioned antibody or antigen-binding fragment may further include at least one of the following additional technical features:
According to the embodiments of the present invention, the antibody comprises:
a heavy chain variable region comprising CDR1, CDR2, CDR3 as shown in SEQ ID NO: 1, 2 and 3 or an amino acid sequence having at least 95% identity to SEQ ID NO: 1, 2 and 3, respectively; and
a light chain variable region comprising CDR1, CDR2, CDR3 as shown in SEQ ID NO: 34, 35 and 36 or an amino acid sequence having at least 95% identity to SEQ ID NO: 34, 35 and 36, respectively;
or, a heavy chain variable region comprising CDR1, CDR2, CDR3 as shown in SEQ ID NO: 4, 5 and 6 or an amino acid sequence having at least 95% identity to SEQ ID NO: 4, 5 and 6, respectively; and
a light chain variable region comprising CDR1, CDR2, CDR3 as shown in SEQ ID NO: 37, 38 and 39 or an amino acid sequence having at least 95% identity to SEQ ID NO: 37, 38 and 39, respectively;
or, a heavy chain variable region comprising CDR1, CDR2, CDR3 as shown in SEQ ID NO: 7, 8 and 9 or an amino acid sequence having at least 95% identity to SEQ ID NO: 7, 8 and 9, respectively; and
a light chain variable region comprising CDR1, CDR2, CDR3 as shown in SEQ ID NO: 40, 41 and 42 or an amino acid sequence having at least 95% identity to SEQ ID NO: 40, 41 and 42, respectively;
or, a heavy chain variable region comprising CDR1, CDR2, CDR3 as shown in SEQ ID NO: 10, 11 and 12 or an amino acid sequence having at least 95% identity to SEQ ID NO: 10, 11 and 12, respectively; and
a light chain variable region comprising CDR1, CDR2, CDR3 as shown in SEQ ID NO: 43, 44 and 45 or an amino acid sequence having at least 95% identity to SEQ ID NO: 43, 44 and 45, respectively;
or, a heavy chain variable region comprising CDR1, CDR2, CDR3 as shown in SEQ ID NO: 13, 14 and 15 or an amino acid sequence having at least 95% identity to SEQ ID NO: 13, 14 and 15, respectively; and
a light chain variable region comprising CDR1, CDR2, CDR3 as shown in SEQ ID NO: 46, 47 and 48 or an amino acid sequence having at least 95% identity to SEQ ID NO: 46, 47 and 48, respectively;
or, a heavy chain variable region comprising CDR1, CDR2, CDR3 as shown in SEQ ID NO: 16, 17 and 18 or an amino acid sequence having at least 95% identity to SEQ ID NO: 16, 17 and 18, respectively; and
a light chain variable region comprising CDR1, CDR2, CDR3 as shown in SEQ ID NO: 49, 50 and 51 or an amino acid sequence having at least 95% identity to SEQ ID NO: 49, 50 and 51, respectively;
or, a heavy chain variable region comprising CDR1, CDR2, CDR3 as shown in SEQ ID NO: 19, 20 and 21 or an amino acid sequence having at least 95% identity to SEQ ID NO: 19, 20 and 21, respectively; and
a light chain variable region comprising CDR1, CDR2, CDR3 as shown in SEQ ID NO: 52, 53 and 54 or an amino acid sequence having at least 95% identity to SEQ ID NO: 52, 53 and 54, respectively;
or, a heavy chain variable region comprising CDR1, CDR2, CDR3 as shown in SEQ ID NO: 22, 23 and 24 or an amino acid sequence having at least 95% identity to SEQ ID NO: 22, 23 and 24, respectively; and
a light chain variable region comprising CDR1, CDR2, CDR3 as shown in SEQ ID NO: 55, 56 and 57 or an amino acid sequence having at least 95% identity to SEQ ID NO: 55, 56 and 57, respectively;
or, a heavy chain variable region comprising CDR1, CDR2, CDR3 as shown in SEQ ID NO: 25, 26 and 27 or an amino acid sequence having at least 95% identity to SEQ ID NO: 25, 26 and 27, respectively; and
a light chain variable region comprising CDR1, CDR2, CDR3 as shown in SEQ ID NO: 58, 59 and 60 or an amino acid sequence having at least 95% identity to SEQ ID NO: 58, 59 and 60, respectively;
or, a heavy chain variable region comprising CDR1, CDR2, CDR3 as shown in SEQ ID NO: 28, 29 and 30 or an amino acid sequence having at least 95% identity to SEQ ID NO: 28, 29 and 30, respectively; and
a light chain variable region comprising CDR1, CDR2, CDR3 as shown in SEQ ID NO: 61, 62 and 63 or an amino acid sequence having at least 95% identity to SEQ ID NO: 61, 62 and 63, respectively;
or, a heavy chain variable region comprising CDR1, CDR2, CDR3 as shown in SEQ ID NO: 31, 32 and 33 or an amino acid sequence having at least 95% identity to SEQ ID NO: 31, 32 and 33, respectively; and
a light chain variable region comprising CDR1, CDR2, CDR3 as shown in SEQ ID NO: 64, 65 and 66 or an amino acid sequence having at least 95% identity to SEQ ID NO: 64, 65 and 66, respectively.

The CDRs of the present invention are defined with reference to the Kabat nomenclature system.

According to the embodiments of the present invention, the antibody or antigen-binding fragment thereof specifically recognizes IL-11.

According to the embodiments of the present invention, the antibody comprises at least one of a heavy chain framework region sequence and a light chain framework region sequence, and at least a part of at least one of the heavy chain framework region sequence and the light chain framework region sequence is derived from at least one of a murine antibody, a human antibody, a primate antibody, or a mutant thereof.

According to the embodiments of the present invention, the heavy chain framework region includes FL1, FL2, FL3 and FL4, and the FL1, FL2, FL3 and FL4 of heavy chain framework region have the amino acid sequences shown in SEQ ID NO: 135, 136, 137 and 138, respectively,
QVQLVQSGAEVKKPGASVKVSCKAS (SEQ ID NO: 135),
LGWVRQAPGX1GLEWX2GH (SEQ ID NO: 136),
NYNEKFKGRX3TX4TX5DTSTSTVYMELSSLRSEDTAVYX6C (SEQ ID NO: 137),
WGQGTLVTVSS (SEQ ID NO: 138),
wherein, X1 is Q or H, X2 is M or I, X3 is V or A, X4 is M or L, X5 is R or A, and X6 is Y or F.

According to the embodiments of the present invention, the light chain framework region includes FL1, FL2, FL3 and FL4, and the FL1, FL2, FL3 and FL4 of light chain framework region have the amino acid sequences shown in SEQ ID NO: 139, 140, 141 and 142, respectively,
EIVLTQSPATLSLSPGERATLSCRAS (SEQ ID NO: 139),
MNWX7QQKPGQAPRLLIY (SEQ ID NO: 140),
NQGSGIPARFSGSGSGTDFTLTISSLEPEDFAX8YX9C (SEQ ID NO: 141),
FGGGTKLEIK (SEQ ID NO: 142),
wherein, X7 is Y or F, X8 is V or M, and X9 is Y or F.

According to the embodiments of the present invention, the humanized antibody proposed by the present invention has a heavy chain variable region with the amino acid sequence shown in any one of SEQ ID NO: 143-146.

QVQLVQSGAEVKKPGASVKVSCKASDYTFTNYWLGWVRQAPGQGLEWM
GHIYPGGGYTNYNEKFKGRVTMTRDTSTSTVYMELSSLRSEDTAVYYCARVRSGNDAL
DFWGQGTLVTVSS (SEQ ID NO: 143).

QVQLVQSGAEVKKPGASVKVSCKASDYTFTNYWLGWVRQAPGQGLEWIG
HIYPGGGYTNYNEKFKGRVTMTADTSTSTVYMELSSLRSEDTAVYFCARVRSGNDALDF

WGQGTLVTVSS (SEQ ID NO: 144).

QVQLVQSGAEVKKPGASVKVSCKASDYTFTNYWLGWVRQAPGQGLEWIG
HIYPGGGYTNYNEKFKGRATLTADTSTSTVYMELSSLRSEDTAVYFCARVRSGNDALDF
WGQGTLVTVSS (SEQ ID NO: 145).

QVQLVQSGAEVKKPGASVKVSCKASDYTFTNYWLGWVRQAPGHGLEWIG
HIYPGGGYTNYNEKFKGRATLTADTSTSTVYMELSSLRSEDTAVYFCARVRSGNDALDF
WGQGTLVTVSS (SEQ ID NO: 146).

According to the embodiments of the present invention, the humanized antibody proposed by the present invention has a light chain variable region with the amino acid sequence shown in any one of SEQ ID NO: 147-150.

EIVLTQSPATLSLSPGERATLSCRASESVDEYGISFMNWYQQKPGQAPRLLIY
SASNQGSGIPARFSGSGSGTDFTLTISSLEPEDFAVYYCQQSKEVPWTFGGGTKLEIK (SEQ
ID NO: 147).

EIVLTQSPATLSLSPGERATLSCRASESVDEYGISFMNWYQQKPGQAPRLLIY
SASNQGSGIPARFSGSGSGTDFTLTISSLEPEDFAVYFCQQSKEVPWTFGGGTKLEIK (SEQ
ID NO: 48).

EIVLTQSPATLSLSPGERATLSCRASESVDEYGISFMNWFQQKPGQAPRLLIY
SASNQGSGIPARFSGSGSGTDFTLTISSLEPEDFAVYFCQQSKEVPWTFGGGTKLEIK (SEQ
ID NO: 149).

EIVLTQSPATLSLSPGERATLSCRASESVDEYGISFMNWFQQKPGQAPRLLIY
SASNQGSGIPARFSGSGSGTDFTLTISSLEPEDFAMYFCQQSKEVPWTFGGGTKLEIK
(SEQ ID NO: 150).

According to the embodiments of the present invention, the heavy chain variable region has the amino acid sequence shown in any one of SEQ ID NO: 67-77.

QIQLVQSGPELKKPGETVKISCKASGYTFTTNGINWVKQAAGKGLKWMGW
INTNTGEPTNAEELKGRFAFSLETSASTAYLQINNLKNEDAAIYFCAREGAFGLDYWGQG
TSVTVSS (SEQ ID NO:67).

QVQLQQSGAELVRPGTSAKISCKASDYTFTNYWLGWLKQRPGHGLEWIGH
IYPGGGYTNYNEKFKGKATLTADTSSSTAYMQLSSLTSEDSAVYFCARVRSGNDALDFW
GQGTSVTVSS (SEQ ID NO:68).

QIQLVQSGPELKKPGETVKISCKASGYTFTNYGMNWVKQAPGKGLKWMG

WINTNTGEPTYAEEIKGRFAFSLETSASTAYLQINNLKNEDTATYFCSREGIYGMDSWGQ
GTSVTVSS (SEQ ID NO:69).

QVQLKESGPGLVAPSQSLSITCTVSGFSLTSYGVHWVRQPPGKGLEWLGIIW
AGGRTDYNSALVSRLNISKDNSKSQVFLKMHSLQTDDTAMYYCAREGGYDYDGDLWT
TGVKEPQSPSPQ (SEQ ID NO:70).

QVQLKQSGPGLVLPSQSLSITCTVSGFSLTSYGVHWFRQSPGTGLEWLGVIW
SGGITDYNATFI.PRLSISKDNSKSQVFFKMNSLQADDTAIYYCARNFDGYYYSIDYWGQ
GTSVTVSS (SEQ ID NO:71).

QVQLKQSGPGLVQPSQSLSITCTVSGFSLTNYGVHWVRQSPGKGLEWLGAI
WRGGSTDYNAAFMSRLSITKDNSKSQVFFKMNSLQPDDTAIYYCAKNLYGPAAMDYW
GQGTSVTVSS (SEQ ID NO:72).

QIQLVQSGPELKKPGETVNISCRASGDTFTNYAVNWVKQAPGKGLKWMGW
IDTYTGDPTYADDLKGRFAFSLETSATTAYLQINNLKNEDTATYFCAGDTWFAYWGQGTL
VTVSA (SEQ ID NO:73).

QVTLKESGPGILQPSQTLSLTCSFSGFSLSSSGMSVGWIRQPSGKGLEWLAHI
WWSDDKSYNPALKSRLTISKDTSNNQVFLKIASVVTADTATYYCAREGSLGYGLDYWG
QGTTLTVSS (SEQ ID NO:74).

EVKLLESGGGLVQPGGSLKLSCAASGFDFRRYWMSWVRQAPGKGLEWIGQ
INPDSSMINYTPSLKDKFIISRDNAKNTLYLQMSEVRSEDTALYYCATYGHHATDSWGQG
TSVTVSS (SEQ ID NO:75).

QVQLQQPGAELVRPGTSVKMSCKASGYTFTSYWMHWVKQRPGQGLEWIG
DIYPGSDSTNYHEKFKSKATLTVDTSSSTAYMQLSSLTSEDSAVYYCALDSSGYGFAYWG
QGTLVTVSA (SEQ ID NO:76).

EVQLVESGGGLVQPGESLKLSCDSNDYEFPSHDMSWVRKTPEKRLELVAAI
NSDGGNTYYPDTMERRFIISRDNTKRTLYLQMSSLRSEDTALHYCARPRPTIGTTATGSS
MSGAQGPRS (SEQ ID NO:77).

According to the embodiments of the present invention, the light chain variable region has the amino acid sequence shown in any one of SEQ ID NO: 78-88.

SIVMTQSPKFLLVSAGDRVIITCKASQSVSNDVVWYQQKPGQSPKLLIYYGS
HRNTGVPTRFTGSGYGTDFTFTISTVQAEDLAVYFCQQDFFSPWTFGGGTKLEIK (SEQ
ID NO:78).

DIVLTQSPASLAVSLGQRATISCRASESVDEYGISFMNWFQQKPGQPPRLLIY
SASNQGSGVPARFTGSGSGTDFSLNILPMEEDDTAMYFCQQSKEVPWTFGGGTKLEIK
(SEQ ID NO:79).

SIVMTQTPKFLLVSAGDRVTITCKASQSVSNDVVWYRQKPGQSPKLLIYYAS
NRYIGVPDRFTGSGYGTDFTFTISTVQAEDLAVYFCQQDYYSPWTFGGGTKLEIK (SEQ
ID NO:80).

DVVMTQSPLSLPVSLGDQASISCRSSQSLVHSNGNTYLHWYLQKPGQSPKL
LIYKVSNRFSGVPDRFSGSGSGTDFTLKISRVEAEDLGVYFCSQSTHVPPDVRWRHQAGN
Q (SEQ ID NO:81).

DIQMTQTTSSLSASLGDRVTISCRASQDISNSLNWYQQKPDGTVKLLIYYTS
RLHSGVPSRFTGSGSGTDYSLTISNLEQEDIATYFCQQGNTLPLTFGAGTKLELK (SEQ ID
NO:82).

DIQMNQSPSSLSASLGDTITITCHANQNIYVWLSWYQQKPGNIPKLLIYEAS
NLHTGVPSRFSGSGSGTGFTLTISSLQPEDIATYYCQQGQSYPYTFGGGTKLEIK (SEQ ID
NO:83).

DIVMSQSPSSLAVSVGEKVTMSCKSSQSLLYSRNQKNRLAWYQQKPGQSPK
LLIYWASTRESGVPDRFTGSGSGTDFTLTISSVKAEDLAVYYCQQYYSYPRTFGGGTNLEI
K (SEQ ID NO:84).

DVLMTQTPLSLPVSLGDQASISCRSSQSIVHTDGNTYLEWYLQKPGQSPKLL
IYKVSNRFSGVPDRFSGSGSGTDFTLKISRVEAEDLGVYYCFQGSHVPWTFGGGTKLEIK
(SEQ ID NO:85).

DIVLTQSPASLAVSLGQRATISCRASESVDSYGNSFMHWYQQKPGQPPKLLI
YRASNLESGIPARFSGSGSRTDFTLTINPVEADDVATYYCQQSNEDPFTFGSGTKLEIK
(SEQ ID NO:86).

NIVMTQTPKFLLVSAGDRVTITRKASQSVSNDVVWYQQKPGQSPKLLMYY
ASYRYTGVPDRFTGSGYGTDFTFTISNVQAEDLAVYFCQQDYSSPFTFGSGTKLEIK (SEQ
ID NO:87).

DIVLTQSPASLAVSLGQRATISCRASQSVSTSTYNYMHWYQQKPGQPPKLLI
KYASNLESGVPARFSGSGSGTDFTLNIHPVEEEDTATYYCQHSWEIPFTFGSGTKLEIK
(SEQ ID NO:88).

According to the embodiments of the present invention, the heavy chain variable region has the amino acid sequence shown in SEQ ID NO: 67, and the light chain variable region has the amino acid sequence shown in SEQ ID NO: 78; or
the heavy chain variable region has the amino acid sequence shown in SEQ ID NO:68, and the light chain variable region has the amino acid sequence shown in SEQ ID NO:79; or
the heavy chain variable region has the amino acid sequence shown in SEQ ID NO:69, and the light chain variable region has the amino acid sequence shown in SEQ ID NO:80; or
the heavy chain variable region has the amino acid sequence shown in SEQ ID NO:70, and the light chain variable region has the amino acid sequence shown in SEQ ID NO:81; or
the heavy chain variable region has the amino acid sequence shown in SEQ ID NO:71, and the light chain variable region has the amino acid sequence shown in SEQ ID NO:82; or
the heavy chain variable region has the amino acid sequence shown in SEQ ID NO:72, and the light chain variable region has the amino acid sequence shown in SEQ ID NO:83; or
the heavy chain variable region has the amino acid sequence shown in SEQ ID NO:73, and the light chain variable region has the amino acid sequence shown in SEQ ID NO:84; or
the heavy chain variable region has the amino acid sequence shown in SEQ ID NO:74, and the light chain variable region has the amino acid sequence shown in SEQ ID NO:85; or
the heavy chain variable region has the amino acid sequence shown in SEQ ID NO:75, and the light chain variable region has the amino acid sequence shown in SEQ ID NO:86; or
the heavy chain variable region has the amino acid sequence shown in SEQ ID NO:76, and the light chain variable region has the amino acid sequence shown in SEQ ID NO:87; or
the heavy chain variable region has the amino acid sequence shown in SEQ ID NO:77, and the light chain variable region has the amino acid sequence shown in SEQ ID NO:88.

According to the embodiments of the present invention, the humanized antibody proposed by the present invention has a heavy chain variable region with the amino acid sequence shown in SEQ ID NO: 143, and a light chain variable region with the amino acid sequence shown in SEQ ID NO: 147; or
the humanized antibody proposed by the present invention has a heavy chain variable region with the amino acid sequence shown in SEQ ID NO: 144, and a light chain variable region with the amino acid sequence shown in SEQ ID NO: 148; or
the humanized antibody proposed by the present invention has a heavy chain variable region with the amino acid sequence shown in SEQ ID NO: 145, and a light chain variable region with the amino acid sequence shown in SEQ ID NO: 149; or
the humanized antibody proposed by the present invention has a heavy chain variable region with the amino acid sequence shown in SEQ ID NO: 146, and a light chain variable region with the amino acid sequence shown in SEQ ID NO: 150.

According to the embodiments of the present invention, the antibody comprises at least one of a heavy chain constant region and a light chain constant region, and at least a part of at least one of the heavy chain constant region and the light chain constant region is derived from at least one of a murine antibody, a human antibody, a primate antibody, or a mutant thereof.

According to the embodiments of the present invention, both the light chain constant region and the heavy chain constant region of the antibody are derived from a human IgG antibody or a mutant thereof. Furthermore, the immunogenicity of the antibody can be effectively reduced.

According to the embodiments of the present invention, the light chain constant region of the antibody is derived from the light chain constant region of human Kappa; the heavy chain constant region is derived from the heavy chain constant region of human IgG1.

According to the embodiments of the present invention, the full-length sequence of the antibody constant region is shown in SEQ ID NO: 89 or 90.






 (SEQ ID NO:89).


 (SEQ
ID NO:90).

Wherein, the full-length sequence of the antibody constant region shown in the above SEQ ID NO: 89 includes an IgG1 heavy chain constant region and a Fc region, wherein the sequence of the IgG1 heavy chain constant region is ASTKGPSVFPLAPSSKSTSGGTAALGCLVKDYFPEPVTVSWNSGALTSGVHTFPAVLQSS GLYSLSSVVTVPSSSLGTQTYICNVNHKPSNTKVDKKV, the sequence of the Fc region is EPKSCDKTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIE KTISKAKGQPREPQVYTLPPSRDELTKNQVSLTCLVKGFYPSDIAVEWESNGQPENNYKT TPPVLDSDGSFFLYSKLTVDKSRWQQGNVFSCSVMHEALHNHYTQKSLSLSPGK; the full-length sequence of the antibody constant region shown in the above SEQ ID NO: 90 is an IgG light chain constant region.

According to the embodiments of the present invention, the heavy chain has the amino acid sequence shown in any one of SEQ ID NO: 91-101 and the light chain has the amino acid sequence shown in any one of SEQ ID NO: 102-112.

QIQLVQSGPELKKPGETVKISCKASGYTFTTNGINWVKQAAGKGLKWMGW
INTNTGEPTNAEELKGRFAFSLETSASTAYLQINNLKNEDAAIYFCAREGAFGLDYWGQG
TSVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTHTCPPCPA
PELLGGPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREPQVY
TLPPSRDELTKNQVSLTCLVKGFYPSDIAVEWESNGQPENNYKTTPPVLDSDGSFFLYSKL
TVDKSRWQQGNVFSCSVMHEALHNHYTQKSLSLSPGK (SEQ ID NO:91).

QVQLQQSGAELVRPGTSAKISCKASDYTFTNYWLGWLKQRPGHGLEWIGH
IYPGGGYTNYNEKFKGKATLTADTSSSTAYMQLSSLTSEDSAVYFCARVRSGNDALDFW
GQGTSVTVSSASTKGPSVFPLAPSSKSTSGGTAALGCLVKDYFPEPVTVSWNSGALTSGV
HTFPAVLQSSGLYSLSSVVTVPSSSLGTQTYICNVNHKPSNTKVDKKVEPKSCDKTHTCP
PCPAPELLGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKAKGQPRE
PQVYTLPPSRDELTKNQVSLTCLVKGFYPSDIAVEWESNGQPENNYKTTPPVLDSDGSFFL
YSKLTVDKSRWQQGNVFSCSVMHEALHNHYTQKSLSLSPGK (SEQ ID NO:92).

QIQLVQSGPELKKPGETVKISCKASGYTFTNYGMNWVKQAPGKGLKWMG
WINTNTGEPTYAEEIKGRFAFSLETSASTAYLQINNLKNEDTATYFCSREGIYGMDSWGQ
GTSVTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKKVEPKSCDKTHTCPPCP
APELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQV

YTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGK (SEQ ID NO:93).

QVQLKESGPGLVAPSQSLSITCTVSGFSLTSYGVHWVRQPPGKGLEWLGIIW
AGGRTDYNSALVSRLNISKDNSKSQVFLKMHSLQTDDTAMYYCAREGGYDYDGDLWT
TGVKEPQSPSPQASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKKVEPKSCDKTHT
CPPCPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQPR
EPQVYTLPPSRDELTKNQVSLTCLVKGFYPSDIAVEWESNGQPENNYKTTPPVLDSDGSF
FLYSKLTVDKSRWQQGNVFSCSVMHEALHNHYTQKSLSLSPGK (SEQ ID NO:94).

QVQLKQSGPGLVLPSQSLSITCTVSGFSLTSYGVHWFRQSPGTGLEWLGVIW
SGGITDYNATFI.PRLSISKDNSKSQVFFKMNSLQADDTAIYYCARNFDGYYYSIDYWGQ
GTSVTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKKVEPKSCDKTHTCPPCP
APELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQV
YTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGK (SEQ ID NO:95).

QVQLKQSGPGLVQPSQSLSITCTVSGFSLTNYGVHWVRQSPGKGLEWLGAI
WRGGSTDYNAAFMSRLSITKDNSKSQVFFKMNSLQPDDTAIYYCAKNLYGPAAMDYW
GQGTSVTVSSASTKGPSVFPLAPSSKSTSGGTAALGCLVKDYFPEPVTVSWNSGALTSGV
HTFPAVLQSSGLYSLSSVVTVPSSSLGTQTYICNVNHKPSNTKVDKKVEPKSCDKTHTCP
PCPAPELLGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKAKGQPRE
PQVYTLPPSRDELTKNQVSLTCLVKGFYPSDIAVEWESNGQPENNYKTTPPVLDSDGSFFL
YSKLTVDKSRWQQGNVFSCSVMHEALHNHYTQKSLSLSPGK (SEQ ID NO:96).

QIQLVQSGPELKKPGETVNISCRASGDTFTNYAVNWVKQAPGKGLKWMGW
IDTYTGDPTYADDLKGRFAFSLETSATTAYLQINNLKNEDTATYFCAGDTWFAYWGQGTL
VTVSAASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTCPPCPAP
ELLGGPSVFLFPPKPKDTLMISRTPEVTCVVVDVSHEDPEVKFNWYVDGVEVHNAKTKP

REEQYNSTYRVVSVLTVLHQDWLNGKEYKCKVSNKALPAPIEKTISKAKGQPREPQVYT
LPPSRDELTKNQVSLTCLVKGFYPSDIAVEWESNGQPENNYKTTPPVLDSDGSFFLYSKLT
VDKSRWQQGNVFSCSVMHEALHNHYTQKSLSLSPGK (SEQ ID NO:97).

QVTLKESGPGILQPSQTLSLTCSFSGFSLSSSGMSVGWIRQPSGKGLEWLAHI
WWSDDKSYNPALKSRLTISKDTSNNQVFLKIASVVTADTATYYCAREGSLGYGLDYWG
QGTTLTVSSASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKSCDKTHTCPPC
PAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQ
VYTLPPSRDELTKNQVSLTCLVKGFYPSDIAVEWESNGQPENNYKTTPPVLDSDGSFFLYS
KLTVDKSRWQQGNVFSCSVMHEALHNHYTQKSLSLSPGK (SEQ ID NO:98).

EVKLLESGGGLVQPGGSLKLSCAASGFDFRRYWMSWVRQAPGKGLEWIGQ
INPDSSMINYTPSLKDKFIISRDNAKNTLYLQMSEVRSEDTALYYCATYGHHATDSWGQG
TSVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTHTCPPCPA
PELLGGPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREPQVY
TLPPSRDELTKNQVSLTCLVKGFYPSDIAVEWESNGQPENNYKTTPPVLDSDGSFFLYSKL
TVDKSRWQQGNVFSCSVMHEALHNHYTQKSLSLSPGK (SEQ ID NO:99).

QVQLQQPGAELVRPGTSVKMSCKASGYTFTSYWMHWVKQRPGQGLEWIG
DIYPGSDSTNYHEKFKSKATLTVDTSSSTAYMQLSSLTSEDSAVYYCALDSSGYGFAYWG
QGTLVTVSAASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKSCDKTHTCPPC
PAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQ
VYTLPPSRDELTKNQVSLTCLVKGFYPSDIAVEWESNGQPENNYKTTPPVLDSDGSFFLYS
KLTVDKSRWQQGNVFSCSVMHEALHNHYTQKSLSLSPGK (SEQ ID NO:100).

EVQLVESGGGLVQPGESLKLSCDSNDYEFPSHDMSWVRKTPEKRLELVAAI
NSDGGNTYYPDTMERRFIISRDNTKRTLYLQMSSLRSEDTALHYCARPRPTIGTTATGSS
MSGAQGPRSASTKGPSVFPLAPSSKSTSGGTAALGCLVKDYFPEPVTVSWNSGALTSGV
HTFPAVLQSSGLYSLSSVVTVPSSSLGTQTYICNVNHKPSNTKVDKKVEPKSCDKTHTCP

PCPAPELLGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKAKGQPRE
PQVYTLPPSRDELTKNQVSLTCLVKGFYPSDIAVEWESNGQPENNYKTTPPVLDSDGSFFL
YSKLTVDKSRWQQGNVFSCSVMHEALHNHYTQKSLSLSPGK (SEQ ID NO:101).

SIVMTQSPKFLLVSAGDRVIITCKASQSVSNDVVWYQQKPGQSPKLLIYYGS
HRNTGVPTRFTGSGYGTDFTFTISTVQAEDLAVYFCQQDFFSPWTFGGGTKLEIKRTVAA
PSVFIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEC (SEQ ID NO:102).

DIVLTQSPASLAVSLGQRATISCRASESVDEYGISFMNWFQQKPGQPPRLLIY
SASNQGSGVPARFTGSGSGTDFSLNILPMEEDDTAMYFCQQSKEVPWTFGGGTKLEIKRT
VAAPSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC (SEQ ID NO:103).

SIVMTQTPKFLLVSAGDRVTITCKASQSVSNDVVWYRQKPGQSPKLLIYYAS
NRYIGVPDRFTGSGYGTDFTFTISTVQAEDLAVYFCQQDYYSPWTFGGGTKLEIKRTVAA
PSVFIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEC (SEQ ID NO:104).

DVVMTQSPLSLPVSLGDQASISCRSSQSLVHSNGNTYLHWYLQKPGQSPKL
LIYKVSNRFSGVPDRFSGSGSGTDFTLKISRVEAEDLGVYFCSQSTHVPPDVRWRHQAGN
QRTVAAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC (SEQ ID
NO:105).

DIQMTQTTSSLSASLGDRVTISCRASQDISNSLNWYQQKPDGTVKLLIYYTS
RLHSGVPSRFTGSGSGTDYSLTISNLEQEDIATYFCQQGNTLPLTFGAGTKLELKRTVAAP
SVFIFPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC (SEQ ID NO:106).

DIQMNQSPSSLSASLGDTITITCHANQNIYVWLSWYQQKPGNIPKLLIYEAS
NLHTGVPSRFSGSGSGTGFTLTISSLQPEDIATYYCQQGQSYPYTFGGGTKLEIKRTVAAP
SVFIFPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC (SEQ ID NO:107).

DIVMSQSPSSLAVSVGEKVTMSCKSSQSLLYSRNQKNRLAWYQQKPGQSPK
LLIYWASTRESGVPDRFTGSGSGTDFTLTISSVKAEDLAVYYCQQYYSYPRTFGGGTNLEI

KRTVAAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC (SEQ ID
NO:108).

DVLMTQTPLSLPVSLGDQASISCRSSQSIVHTDGNTYLEWYLQKPGQSPKLL
IYKVSNRFSGVPDRFSGSGSGTDFTLKISRVEAEDLGVYYCFQGSHVPWTFGGGTKLEIK
RTVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC (SEQ ID NO:109).

DIVLTQSPASLAVSLGQRATISCRASESVDSYGNSFMHWYQQKPGQPPKLLI
YRASNLESGIPARFSGSGSRTDFTLTINPVEADDVATYYCQQSNEDPFTFGSGTKLEIKRTV
AAPSVFIFPPSDEQLKSGTASVVCLLNNFYPREAKVQWKVDNALQSGNSQESVTEQDSK
DSTYSLSSTLTLSKADYEKHKVYACEVTHQGLSSPVTKSFNRGEC (SEQ ID NO:110).

NIVMTQTPKFLLVSAGDRVTITRKASQSVSNDVVWYQQKPGQSPKLLMYY
ASYRYTGVPDRFTGSGYGTDFTFTISNVQAEDLAVYFCQQDYSSPFTFGSGTKLEIKRTV
AAPSVFIFPPSDEQLKSGTASVVCLLNNFYPREAKVQWKVDNALQSGNSQESVTEQDSK
DSTYSLSSTLTLSKADYEKHKVYACEVTHQGLSSPVTKSFNRGEC (SEQ ID NO:111).

DIVLTQSPASLAVSLGQRATISCRASQSVSTSTYNYMHWYQQKPGQPPKLLI
KYASNLESGVPARFSGSGSGTDFTLNIHPVEEEDTATYYCQHSWEIPFTFGSGTKLEIKRT
VAAPSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC (SEQ ID NO:112).

Wherein, in this application, the antibody composed of the above SEQ ID NO: 91 and 102 is referred to as 3-6A4, the antibody composed of the above SEQ ID NO: 92 and 103 is referred to as 5-20E11-5F2, the antibody composed of the above SEQ ID NO: 93 and 104 is referred to as 3-5B2-2G4, the antibody composed of the above SEQ ID NO: 94 and 105 is referred to as 3-5B2-7A4, the antibody composed of the above SEQ ID NO: 95 and 106 is referred to as 3-2B5-3D2, the antibody composed of the above SEQ ID NO: 96 and SEQ ID NO:107 is referred to as 3-7C9-1A6, the antibody composed of the above SEQ ID NO: 97 and SEQ ID NO:108 is referred to as 3-9E1-3A2, the antibody composed of the above SEQ ID NO: 98 and SEQ ID NO:109 is referred to as 3-15D5-3A6, the antibody composed of the above SEQ ID NO: 99 and SEQ ID NO:110 is referred to as 3-15D5-6A1, the antibody composed of the above SEQ ID NO: 100 and SEQ ID NO:111 is referred to as 5-20E11-1A2, the antibody composed of the above SEQ ID NO: 101 and SEQ ID NO:112 is referred to as 5-21G2-1D3.

According to the embodiments of the present invention, the humanized antibody proposed by the present invention has a heavy chain with the amino acid sequence shown in any one of SEQ ID NO: 151-154 and a light chain with the amino acid sequence shown in any one of SEQ ID NO: 155-158.

QVQLVQSGAEVKKPGASVKVSCKASDYTFTNYWLGWVRQAPGQGLEWM
GHIYPGGGYTNYNEKFKGRVTMTRDTSTSTVYMELSSLRSEDTAVYYCARVRSGNDAL
DFWGQGTLVTVSSASTKGPSVFPLAPSSKSTSGGTAALGCLVKDYFPEPVTVSWNSGALT
SGVHTFPAVLQSSGLYSLSSVVTVPSSSLGTQTYICNVNHKPSNTKVDKKVEPKSCDKTH
TCPPCPAPELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISKAKGQP
REPQVYTLPPSRDELTKNQVSLTCLVKGFYPSDIAVEWESNGQPENNYKTTPPVLDSDGS
FFLYSKLTVDKSRWQQGNVFSCSVMHEALHNHYTQKSLSLSPGK (SEQ ID NO:151).

QVQLVQSGAEVKKPGASVKVSCKASDYTFTNYWLGWVRQAPGQGLEWIG
HIYPGGGYTNYNEKFKGRVTMTADTSTSTVYMELSSLRSEDTAVYFCARVRSGNDALDF
WGQGTLVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKTHTC
PPCPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQPR
EPQVYTLPPSRDELTKNQVSLTCLVKGFYPSDIAVEWESNGQPENNYKTTPPVLDSDGSF
FLYSKLTVDKSRWQQGNVFSCSVMHEALHNHYTQKSLSLSPGK (SEQ ID NO:152).

QVQLVQSGAEVKKPGASVKVSCKASDYTFTNYWLGWVRQAPGQGLEWIG
HIYPGGGYTNYNEKFKGRATLTADTSTSTVYMELSSLRSEDTAVYFCARVRSGNDALDF
WGQGTLVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKTHTC
PPCPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQPR
EPQVYTLPPSRDELTKNQVSLTCLVKGFYPSDIAVEWESNGQPENNYKTTPPVLDSDGSF
FLYSKLTVDKSRWQQGNVFSCSVMHEALHNHYTQKSLSLSPGK (SEQ ID NO:153).

QVQLVQSGAEVKKPGASVKVSCKASDYTFTNYWLGWVRQAPGHGLEWIG
HIYPGGGYTNYNEKFKGRATLTADTSTSTVYMELSSLRSEDTAVYFCARVRSGNDALDF
WGQGTLVTVSSASTKGPSVFPLAPSSKSTSGGTAALGCLVKDYFPEPVTVSWNSGALTSG

VHTFPAVLQSSGLYSLSSVVTVPSSSLGTQTYICNVNHKPSNTKVDKKVEPKSCDKTHTC
PPCPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQPR
EPQVYTLPPSRDELTKNQVSLTCLVKGFYPSDIAVEWESNGQPENNYKTTPPVLDSDGSF
FLYSKLTVDKSRWQQGNVFSCSVMHEALHNHYTQKSLSLSPGK (SEQ ID NO:154).

EIVLTQSPATLSLSPGERATLSCRASESVDEYGISFMNWYQQKPGQAPRLLIY
SASNQGSGIPARFSGSGSGTDFTLTISSLEPEDFAVYYCQQSKEVPWTFGGGTKLEIKRTV
AAPSVFIFPPSDEQLKSGTASVVCLLNNFYPREAKVQWKVDNALQSGNSQESVTEQDSK
DSTYSLSSTLTLSKADYEKHKVYACEVTHQGLSSPVTKSFNRGEC (SEQ ID NO:155).

EIVLTQSPATLSLSPGERATLSCRASESVDEYGISFMNWYQQKPGQAPRLLIY
SASNQGSGIPARFSGSGSGTDFTLTISSLEPEDFAVYFCQQSKEVPWTFGGGTKLEIKRTVA
APSVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC (SEQ ID NO:156).

EIVLTQSPATLSLSPGERATLSCRASESVDEYGISFMNWFQQKPGQAPRLLIY
SASNQGSGIPARFSGSGSGTDFTLTISSLEPEDFAVYFCQQSKEVPWTFGGGTKLEIKRTVA
APSVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC (SEQ ID NO:157).

EIVLTQSPATLSLSPGERATLSCRASESVDEYGISFMNWFQQKPGQAPRLLIY
SASNQGSGIPARFSGSGSGTDFTLTISSLEPEDFAMYFCQQSKEVPWTFGGGTKLEIKRTV
AAPSVFIFPPSDEQLKSGTASVVCLLNNFYPREAKVQWKVDNALQSGNSQESVTEQDSK
DSTYSLSSTLTLSKADYEKHKVYACEVTHQGLSSPVTKSFNRGEC (SEQ ID NO:158).

According to the embodiments of the present invention, the antibody is a single-chain antibody, a multimeric antibody, a CDR-grafted antibody or a small molecule antibody.

According to the embodiments of the present invention, the single-chain antibody comprises a heavy chain variable region with the amino acid sequence shown in any one of SEQ ID NO: 67-77 and a light chain variable region with the amino acid sequence shown in any one of SEQ ID NO: 78-88, wherein the C-terminus of the heavy chain variable region is connected to the N-terminus of the light chain variable region through a connecting peptide linker, or the C-terminus of the light chain variable region is connected to the N-terminus of the heavy chain variable region through a connecting peptide linker. It should be noted that the "connecting peptide linker" of the single-chain antibody described in this application is a connecting peptide used to connect the heavy chain variable region and light chain variable region of the antibody, which can be a commonly used connecting peptide linker for preparing single-chain antibodies, and can also be a connecting peptide Linker modified by scientific researchers.

According to the embodiments of the present invention, the small molecule antibody comprises at least one of Fab antibody, Fv antibody, single domain antibody and minimum recognition unit.

In the second aspect of the present invention, the present invention provides a nucleic acid molecule. According to the embodiments of the present invention, the nucleic acid molecule encodes the aforementioned antibody or antigen-binding fragment thereof. The antibody or antigen-binding fragment encoded by the nucleic acid molecule according to the embodiments of the present invention can specifically target and bind to IL-11, and block the binding of IL-11 and IL-11 receptor.

According to the embodiments of the present invention, the above-mentioned nucleic acid molecule may further include at least one of the following additional technical features:

According to the embodiments of the present invention, the nucleic acid molecule is DNA.

According to the embodiments of the present invention, the nucleic acid molecule has the nucleotide sequence shown in any one of SEQ ID NO: 113-123 or has the nucleotide sequence shown in any one of SEQ ID NO: 124-134.

CAGATTCAATTGGTACAAAGCGGTCCCGAGCTCAAGAAACCTGGGGAGA
CTGTGAAAATTTCCTGTAAGGCCTCTGGCTACACCTTCACCACTAACGGTATCAACTG
GGTGAAGCAGGCTGCAGGAAAGGGGCTCAAGTGGATGGGATGGATCAACACTAATA
CTGGAGAACCCACCAATGCTGAGGAACTTAAAGGCAGATTCGCCTTCTCTCTTGAGA
CTTCAGCCAGCACCGCCTACCTCCAAATTAACAATCTGAAAAATGAGGATGCCGCTAT
CTATTTTTGTGCCCGAGAGGGTGCATTCGGTCTTGATTACTGGGGTCAGGGCACCTCT
GTGACAGTGTCTAGCGCCTCAACTAAGGGGCCCAGTGTTTCCCCCTTGCCCCTTCCT
CAAAATCAACTAGCGGAGGCACAGCCGCTCTTGGCTGTCTCGTGAAAGATTACTTCC
CCGAGCCAGTGACAGTCTCTTGGAACAGCGGGGCACTCACCTCCGGCGTTCACACTT
TTCCCGCAGTCCTCCAGTCTAGCGGATTGTATAGCCTGTCTTCTGTTGTCACAGTGCCA

TCCTCTAGCCTTGGCACCCAGACCTACATTTGTAATGTGAATCATAAGCCTTCAAACA
CAAAAGTCGATAAAAAAGTGGAGCCAAAGAGCTGTGACAAGACTCACACTTGCCCT
CCATGCCCTGCCCCAGAACTCCTTGGCGGACCTAGCGTCTTCCTGTTTCCTCCTAAGC
CAAAGGACACCCTGATGATCTCACGCACTCCTGAAGTGACCTGCGTTGTGGTAGACG
TGAGCCACGAGGATCCAGAGGTGAAGTTCAACTGGTATGTGGATGGAGTCGAGGTGC
ATAATGCCAAAACAAAACCTAGGGAGGAGCAGTACAACTCTACCTACCGGGTGGTGT
CTGTTTTGACCGTTCTGCACCAAGACTGGTTGAATGGCAAGGAGTACAAGTGCAAGG
TGTCAAACAAAGCACTCCCAGCCCCTATAGAGAAGACCATCAGCAAAGCTAAGGGG
CAGCCAAGAGAACCACAGGTTTACACCCTGCCCCCCTCACGGGATGAACTGACAAA
GAACCAGGTGTCTCTGACTTGCCTCGTGAAAGGATTCTACCCTTCAGATATCGCAGTG
GAGTGGGAGTCTAACGGCCAACCCGAGAATAATTACAAGACAACTCCCCCAGTCCTG
GACAGTGATGGGTCTTTTTTCCTGTATTCTAAGCTCACTGTTGACAAAAGCCGCTGGC
AGCAGGGTAATGTATTCTCCTGCTCCGTCATGCACGAAGCCCTGCACAACCACTATAC
CCAGAAGTCTCTGAGCTTGTCACCCGGCAAG (SEQ ID NO:113).

CAAGTACAGTTGCAGCAGTCTGGAGCTGAGCTGGTTAGACCTGGAACCA
GTGCCAAGATCTCCTGCAAAGCTAGTGATTATACTTTCACAAACTACTGGCTCGGTTG
GCTGAAACAAAGACCCGGGCACGGTCTGGAATGGATAGGCCACATTTACCCAGGCGG
CGGATACACCAACTATAACGAGAAGTTTAAGGGAAAGGCTACTCTGACAGCCGACAC
CTCATCCAGCACAGCCTACATGCAGCTTAGTAGTTTGACCAGTGAGGATTCCGCAGTG
TACTTCTGTGCACGTGTGAGGTCAGGGAATGACGCCCTGGACTTCTGGGGCCAGGGT
ACTTCAGTGACAGTGTCCTCCGCTAGCACTAAGGGACCAAGCGTGTTTCCTCTGGCT
CCAAGCTCTAAATCAACCTCAGGCGGAACCGCCGCTTTGGGATGTCTTGTGAAGGAT
TACTTCCCTGAGCCTGTCACCGTCTCCTGGAATAGCGGCGCCCTGACTTCCGGGGTGC
ATACATTCCCTGCCGTATTGCAGTCAAGCGGCCTGTATAGCCTTAGCTCTGTTGTAACT
GTGCCATCTTCTTCCCTTGGCACACAGACCTACATCTGTAACGTGAACCATAAGCCAT
CCAATACAAAAGTTGATAAGAAGGTGGAGCCAAAATCATGTGACAAAACTCATACAT
GCCCTCCTTGTCCAGCACCTGAGCTGTTGGGCGGACCAAGTGTCTTTCTGTTCCCCCC
AAAGCCAAAGGACACACTGATGATCAGTCGCACACCAGAAGTGACTTGCGTTGTTGT
CGATGTGAGCCACGAAGACCCTGAGGTTAAGTTTAACTGGTACGTGGATGGTGTGGA
GGTGCACAATGCCAAGACTAAGCCACGTGAAGAACAGTATAACTCCACCTATCGTGT
GGTCTCTGTCCTGACAGTACTGCACCAGGACTGGCTCAATGGAAAAGAGTACAAGTG

CAAGGTTAGTAACAAGGCCTTGCCCGCTCCTATCGAGAAAACTATCAGTAAGGCAAA
GGGACAGCCAAGAGAACCACAGGTTTACACCCTGCCACCTAGCCGGGACGAATTGA
CTAAAAACCAAGTGAGTCTCACATGCCTCGTCAAGGGATTCTACCCATCTGACATTGC
CGTAGAGTGGGAGTCCAATGGGCAGCCCGAGAATAATTATAAGACTACTCCACCTGTC
CTGGATTCTGATGGATCCTTCTTTTGTATTCCAAGCTTACCGTGGACAAAAGCAGGT
GGCAGCAGGGCAATGTCTTTTCATGCAGCGTGATGCACGAAGCACTTCACAATCACT
ACACACAGAAGAGTCTGTCTCTCAGTCCTGGTAAG (SEQ ID NO:114).

CAAATTCAGCTGGTGCAGTCCGGCCCAGAACTTAAGAAACCCGGTGAAA
CCGTGAAGATATCCTGCAAAGCTTCAGGCTATACCTTTACCAACTATGGCATGAATTG
GGTGAAGCAAGCTCCCGGGAAGGGCCTTAAATGGATGGGCTGGATCAACACCAACA
CAGGTGAGCCAACCTATGCCGAAGAAATCAAAGGACGCTTTGCTTTCTCTCTCGAAA
CTAGCGCAAGTACCGCTTATTTGCAGATCAATAATCTGAAAAACGAGGACACCGCTAC
CTACTTTTGCTCAAGAGAGGGCATTTACGGCATGGATTCTTGGGGCCAGGGAACATCT
GTCACCGTCTCATCTGCATCTACCAAGGGCCCTAGTGTGTTCCCACTGGCCCCTTCAT
CCAAGTCCACTTCTGGAGGAACAGCCGCCTTGGGCTGTCTCGTTAAGGACTACTTCC
CCGAGCCTGTTACCGTGTCCTGGAACAGTGGCGCCCTGACCTCCGGTGTTCACACTT
TCCCCGCTGTGTTGCAGTCCTCTGGCCTTTATTCCCTTTCCTCTGTAGTGACAGTGCCC
AGTTCCAGTCTGGGTACACAGACCTACATTTGCAATGTCAACCATAAACCAAGTAATA
CCAAGGTAGATAAAAAGGTAGAGCCTAAGTCATGCGATAAAACTCACACATGTCCAC
CATGTCCTGCTCCAGAACTCTTGGGCGGTCCAAGTGTCTTTTTGTTCCCACCAAAACC
TAAGGATACCCTCATGATCTCTAGGACCCCCGAGGTCACATGTGTGGTGGTCGACGTT
AGTCACGAGGATCCCGAGGTTAAATTTAACTGGTATGTCGATGGGGTTGAAGTCCAC
AATGCCAAAACCAAACCAAGAGAGGAACAGTACAATTCCACTTACCGTGTGGTTTCA
GTGCTGACCGTCCTTCATCAGGACTGGCTCAACGGGAAGGAGTACAAGTGCAAGGT
CAGTAACAAGGCTCTTCCAGCCCCCATCGAAAAGACTATTTCCAAAGCCAAGGGACA
ACCAAGAGAGCCACAAGTGTACACTCTTCCCCCCAGCAGGGACGAACTCACAAAGA
ATCAGGTTTCTCTCACCTGTCTGGTTAAGGGCTTTTACCCCAGCGACATCGCAGTTGA
GTGGGAGAGCAATGGCCAGCCTGAGAATAACTATAAGACAACACCCCCCGTGCTGGA
TTCTGACGGCAGCTTTTTCTTGTACTCTAAACTCACTGTAGACAAATCCAGGTGGCAG
CAAGGCAACGTGTTTTCCTGCTCTGTCATGCACGAGGCATTGCACAATCACTATACTC
AGAAATCACTTAGCCTGTCCCCCGGTAAG (SEQ ID NO:115).

CAAGTACAATTGAAAGAGAGTGGACCAGGACTTGTGGCCCCTAGCCAGT
CTCTGTCAATAACCTGTACTGTCAGTGGATTTTCCCTTACCAGCTATGGGGTACACTGG
GTTAGGCAGCCACCTGGTAAAGGACTTGAGTGGCTGGGCATTATTTGGCCGGAGGG
CGGACAGACTATAATTCTGCACTGGTATCCAGACTCAATATCAGTAAGGACAACAGCA
AAAGTCAAGTGTTTCTGAAAATGCATTCACTTCAAACCGATGACACCGCTATGTACTA
CTGTGCTAGGGAAGGCGGTTATGACTATGATGGAGATCTCTGGACTACCGGAGTCAA
GGAGCCTCAAAGCCCCAGTCCTCAGGCCTCCACAAAGGGCCTAGTGTTTCCCCCT
GGCCCCATCATCTAAGAGTACATCAGGAGGAACAGCAGCCCTTGGATGCCTGGTGAA
AGACTACTTTCCAGAACCCGTTACCGTGTCCTGGAACTCAGGCGCACTTACCTCTGG
CGTCCATACATTTCCCGCCGTATTGCAGAGCAGCGGACTGTACTCATTGTCATCCGTG
GTGACAGTGCCTAGTAGTTCTCTTGGTACACAGACCTACATATGTAACGTGAATCATA
AACCCTCAAATACTAAAGTCGACAAGAAGGTTGAGCCCAAGAGCTGTGATAAAACCC
ACACTTGTCCCCCATGCCCAGCACCTGAACTGCTCGGAGGCCCATCCGTCTTTCTCTT
CCCTCCTAAGCCTAAGGATACACTTATGATTTCACGGACCCCTGAAGTGACATGTGTG
GTCGTTGATGTGTCCCATGAGGACCCAGAAGTTAAATTTAACTGGTATGTCGATGGCG
TGGAGGTGCACAACGCCAAGACAAAGCCCAGGGAAGAACAGTATAATTCAACATAC
CGCGTCGTTAGCGTGCTGACAGTTCTGCATCAGGATTGGCTTAACGGTAAAGAGTAC
AAGTGCAAGGTCTCCAACAAAGCTCTGCCTGCACCTATTGAAAAAACTATATCCAAG
GCTAAGGGCCAGCCTCGCGAGCCTCAGGTATATACACTGCCACCCAGCAGGGACGAA
CTGACAAAGAATCAGGTCAGTCTGACTTGCCTCGTCAAGGGATTCTACCCTTCAGAC
ATCGCCGTGGAGTGGGAGTCAAACGGTCAGCCCGAGAACAACTACAAGACAACACC
CCCTGTACTGGACTCCGACGGCTCCTTTTTCCTGTATTCTAAGCTGACTGTCGATAAAT
CTCGCTGGCAGCAGGGAAACGTCTTTTCCTGCTCTGTGATGCACGAGGCTCTGCACA
ATCACTATACTCAGAAGTCACTGTCCTTGTCTCCCGGAAAG (SEQ ID NO:116).

CAGGTACAATTGAAACAAAGTGGGCCAGGTCTTGTCCTTCCTTCCCAGTC
TCTGAGTATTACCTGTACCGTCAGTGGATTTAGCCTTACAAGCTATGGAGTCCATTGGT
TCAGGCAATCCCCAGGCACAGGCCTGGAATGGTTGGGGGTCATCTGGAGTGGTGGTA
TCACCGATTACAATGCAACCTTTATCCCCCGTCTGTCCATATCAAAGGACAACTCTAA
GTCCCAGGTGTTTTTCAAAATGAACTCTCTCCAGGCAGATGACACAGCCATATATTAC
TGTGCTCGTAACTTCGACGGCTACTACTATTCCATAGATTATTGGGGCCAGGGCACTTC
AGTCACCGTCAGTAGCGCCAGCACTAAGGGTCCTTCCGTTTTTCCCCTTGCTCCTTCT

TCAAAGAGCACAAGTGGGGGGACTGCAGCTTTGGGCTGCTTGGTGAAGGATTATTTT
CCTGAGCCTGTCACAGTCTCTTGGAATAGCGGGGCACTCACAAGCGGTGTTCATACC
TTTCCAGCCGTGCTGCAGAGTTCTGGTCTGTATAGTCTCTCCTCAGTGGTGACCGTAC
CTTCTTCCTCTCTGGGCACCCAGACATACATATGCAATGTTAATCACAAGCCCTCCAA
CACAAAAGTGGATAAAAAAGTTGAGCCTAAGAGTTGTGACAAAACTCACACATGTCC
TCCTTGCCCAGCTCCCGAACTCCTTGGAGGACCAAGTGTTTTTTTGTTTCCTCCTAAG
CCCAAGGACACCCTGATGATTTCTAGAACTCCAGAGGTAACATGTGTGGTCGTGGAC
GTCAGCCATGAGGACCCCGAAGTAAAATTTAATTGGTACGTGGACGGGGTGGAGGTG
CATAACGCCAAGACCAAACCCCGAGAGGAGCAGTATAATAGCACCTACAGAGTCGTG
AGTGTTTTGACTGTCCTGCATCAAGATTGGCTCAACGGAAAGGAGTACAAGTGCAAG
GTAAGTAATAAAGCCCTGCCAGCACCCATAGAGAAAACCATCAGCAAAGCAAAAGG
GCAGCCAAGAGAGCCACAGGTTTATACACTCCCTCCATCTCGGGACGAGTTGACTAA
GAACCAGGTTAGTCTGACATGTCTGGTGAAAGGCTTTTACCCCAGCGATATAGCTGTA
GAGTGGGAAAGCAACGGGCAGCCTGAGAACAACTATAAGACCACTCCCCCCGTTCT
GGATAGCGACGGTAGTTTCTTTCTTTATTCCAAGCTGACTGTGGATAAATCCCGATGGC
AGCAGGGAAATGTCTTTTCCTGTTCCGTCATGCACGAAGCTCTCCATAATCACTACAC
TCAGAAATCTCTGTCCCTGAGTCCCGGTAAA (SEQ ID NO:117).

CAAGTTCAACTTAAACAGAGTGGTCCTGGCCTGGTACAGCCAAGCCAGT
CCCTGTCCATAACCTGTACAGTCTCAGGCTTTAGCCTTACTAACTATGGCGTCCACTGG
GTTCGGCAGTCTCCTGGTAAGGGACTTGAATGGCTCGGAGCTATTTGGAGGGGAGGG
TCCACCGACTATAATGCAGCATTCATGTCCCGGCTTTCTATTACCAAGGATAACAGCAA
AAGTCAGGTGTTCTTTAAGATGAACTCCCTGCAGCCCGACGATACAGCCATCTACTAC
TGTGCCAAAAACCTGTATGGACCAGCTGCCATGGACTATTGGGGCCAGGGGACCTCC
GTCACAGTTAGCAGCGCTTCTACTAAGGGCCCCTCTGTGTTTCCACTGGCACCATCCT
CTAAGTCAACCAGTGGGGGCACTGCAGCTTTGGGCTGTCTGGTCAAGGATTACTTTC
CAGAACCTGTCACCGTTTCCTGGAATTCCGGCGCCTTGACTTCTGGTGTGCACACCTT
CCCAGCTGTCCTGCAGTCCAGTGGACTGTATTCACTGAGCTCCGTGGTCACAGTGCC
TTCAAGTAGCCTGGGCACCCAGACCTATATCTGTAACGTAAACCACAAGCCTTCTAAC
ACCAAGGTGGACAAAAAGGTGGAACCAAAAAGCTGTGACAAAACTCATACATGCCC
ACCTTGTCCAGCTCCAGAGCTGCTGGGTGGACCATCTGTGTTCTTGTTTCCCCCTAAG
CCCAAAGACACCCTGATGATTTCACGGACCCCTGAGGTGACATGTGTCGTTGTGGAC

GTTAGTCACGAGGATCCTGAAGTAAAATTCAATTGGTACGTCGACGGCGTCGAAGTT
CACAATGCCAAGACAAAGCCCCGAGAGGAACAGTACAATAGTACATACCGAGTCGTG
AGCGTGTTGACAGTTCTCCACCAGGATTGGCTCAATGGCAAAGAGTACAAATGTAAG
GTATCAAATAAAGCCCTCCCCGCTCCCATAGAGAAGACAATCTCAAAGGCCAAAGGG
CAACCCAGGGAGCCTCAAGTCTACACCCTTCCCCCATCTCGCGACGAGCTCACCAAG
AATCAGGTATCATTGACATGTCTGGTCAAAGGATTTATCCTAGCGATATCGCCGTAGA
GTGGGAGTCTAATGGCCAGCCCGAGAATAATTACAAAACCACTCCTCCTGTGCTGGAT
TCTGATGGATCTTTCTTCCTGTATAGTAAGTTGACAGTCGATAAGTCCCGGTGGCAAC
AGGGTAATGTTTTCAGCTGTTCCGTTATGCATGAGGCTCTCCACAACCATTACACCCA
AAAGAGTCTTTCTCTTTCCCCCGGCAAG (SEQ ID NO:118).

CAGATTCAGCTGGTGCAGTCCGGTCCTGAGTTGAAAAAGCCAGGAGAAA
CTGTCAACATCTCTTGCCGCGCCAGCGGGACACATTCACCAACTACGCCGTGAACT
GGGTGAAGCAGGCTCCCGGTAAGGGGCTCAAGTGGATGGGCTGGATAGACACCTAC
ACAGGAGATCCTACCTACGCAGACGACTTGAAGGGCAGGTTCGCCTTCTCTCTTGAG
ACCTCCGCCACAACTGCTTATCTTCAGATCAACAATTTGAAAAACGAGGATACCGCCA
CTTACTTCTGTGCTGGGGATACCTGGTTCGCTTACTGGGGGCAGGGAACTTTGGTCAC
CGTGAGCGCTGCTTCCACCAAGGGGCCCAGCGTCTTCCCTCTCGCCCCTTCTAGCAA
GAGCACCAGTGGTGGAACCGCTGCCCTTGGGTGCCTCGTGAAGGATTATTTCCCTGA
ACCTGTGACTGTATCTTGGAATAGCGGTGCACTCACCTCCGGTGTGCACACTTTTCCA
GCTGTGCTGCAGTCTAGCGGGCTGTATAGCCTTAGCTCCGTGGTGACCGTGCCTTCTT
CTTCTTTGGGTACCCAGACCTACATCTGCAACGTGAACCATAAGCCATCCAATACTAA
GGTCGACAAGAAAGTGGAGCCCAAATCCTGCGATAAAACCCATACTTGTCCCCCATG
CCCAGCTCCAGAACTGCTCGGAGGGCCCAGTGTGTTTCTTTTCCCCCCAAAGCCTAA
GGACACTCTCATGATATCCCGAACCCCAGAGGTGACATGCGTCGTTGTGGATGTGTCA
CATGAAGATCCAGAGGTCAAGTTCAACTGGTATGTCGATGGAGTCGAGGTCCACAAT
GCTAAGACAAAACCAAGAGAGGAGCAGTATAACAGTACCTACCGAGTGGTCTCCGTG
CTGACTGTTCTTCACCAAGATTGGCTCAATGGTAAAGAGTACAAATGCAAAGTATCCA
ACAAGGCCCTTCCTGCACCAATCGAAAAAACTATTTCCAAAGCAAAGGGCCAGCCCA
GGGAGCCACAGGTCTACACACTGCCCCCAAGCAGGGATGAACTCACCAAGAACCAA
GTGTCACTTACCTGCCTGGTTAAGGGATTCTACCCCTCCGATATTGCAGTAGAATGGG
AGTCTAACGGTCAGCCTGAGAATAACTACAAGACCACCCCCCCCGTGCTCGATAGCG

ATGGTTCATTCTTCCTGTATAGCAAGCTGACCGTCGATAAGAGTAGGTGGCAACAAGG
GAATGTGTTCAGTTGCTCTGTTATGCACGAAGCCCTCCACAACCATTACACACAAAAG
TCCCTTTCCTTGTCTCCCGGTAAG (SEQ ID NO:119).

CAGGTAACACTTAAGGAGAGCGGTCCAGGTATACTCCAGCCTAGTCAGA
CACTGAGCCTTACATGCAGCTTTAGTGGGTTCAGCCTGTCCAGTTCTGGCATGTCAGT
AGGCTGGATCCGACAACCTAGCGGAAAGGGCCTGGAGTGGCTGGCTCATATCTGGTG
GTCAGACGACAAGTCCTATAACCCAGCCCTCAAATCCCGTCTGACCATCTCAAAAGA
CACCAGTAATAATCAGGTTTTCCTTAAAATCGCCTCTGTGGTCACTGCAGATACAGCA
ACCTACTACTGTGCACGCGAGGGCTCTTTGGGCTATGGACTGGACTACTGGGGTCAG
GGTACCACTCTGACTGTAAGCAGTGCTTCAACTAAGGGACCTAGCGTGTTTCCACTG
GCACCCAGTTCTAAATCTACTTCTGGGGGAACTGCTGCTCTGGGATGCCTGGTGAAG
GATTACTTTCCAGAGCCCGTCACTGTGAGTTGGAATAGCGGTGCCTTGACCTCCGGA
GTGCACACCTTCCCAGCTGTGCTTCAATCCTCAGGCTTGTATTCTCTTTCCTCCGTGGT
GACCGTGCCATCCTCCTCTCGGGACCCAGACTTACATATGTAATGTAAACCACAAG
CCCTCTAACACAAAGGTAGATAAGAAGGTCGAGCCAAAGTCCTGTGATAAAACACAT
ACATGTCCCCCTTGTCCCGCTCCAGAACTGCTTGGCGGACCCTCTGTGTTCCTGTTCC
CACCTAAGCCAAAAGACACTCTTATGATTTCCAGGACTCCAGAGGTTACATGCGTTGT
GGTGGACGTAAGCCATGAAGACCCTGAGGTGAAATTCAATTGGTATGTGGACGGTGT
GGAGGTCCATAACGCTAAGACTAAGCCCAGGGAGGAACAGTACAACTCAACATATCG
AGTCGTCTCTGTCCTGACAGTGCTCCACCAGGATTGGCTGAACGGAAAGGAATACAA
ATGCAAGGTGAGTAATAAAGCCCTGCCAGCTCCCATCGAAAAAACAATCAGCAAGGC
TAAGGGTCAGCCCCGCGAACCACAGGTCTACACTCTGCCACCAAGTCGGGATGAATT
GACTAAGAATCAGGTCTCCCTGACCTGTCTGGTGAAGGGTTTTTATCCCTCCGACATC
GCAGTGGAGTGGGAGAGCAACGGGCAGCCTGAGAATAACTATAAGACAACTCCACC
CGTCCTCGATTCTGACGGGTCTTTTTTCTGTACAGCAAACTGACTGTGGACAAGTCT
CGGTGGCAGCAAGGTAACGTCTTCAGCTGCTCCGTTATGCACGAAGCCCTCCACAAC
CACTACACTCAGAAATCTCTGTCCTTGAGTCCTGGAAAG (SEQ ID NO:120).

GAAGTTAAACTCCTCGAATCTGGAGGAGGGTTGGTTCAGCCAGGTGGTT
CTTTGAAGCTGTCTTGTGCAGCCTCTGGGTTTGACTTCCGAAGATATTGGATGAGTTG
GGTGCGTCAGGCACCAGGGAAAGGTCTTGAATGGATCGGGCAGATTAACCCCGACA
GTTCAATGATTAACTATACACCTTCTTTGAAAGATAAATTTATTATATCCCGTGACAACG

CTAAAAACACCTTGTATCTGCAGATGAGTGAGGTGAGATCCGAAGACACTGCTCTTT
ACTACTGTGCCACCTATGGACACCACGCTACAGACTCCTGGGGCCAGGGTACCAGTG
TGACAGTTTCCAGTGCCAGCACCAAGGGCCCATCCGTTTTCCCCCTGGCTCCCTCTTC
TAAATCTACTTCAGGTGGTACTGCAGCACTTGGATGTTTGGTGAAGGACTACTTCCCT
GAGCCAGTCACCGTGAGTTGGAACTCAGGCGCTCTTACATCCGGTGTCCACACATTT
CCAGCCGTGCTGCAGAGCTCAGGACTCTATTCTCTCAGCTCCGTCGTGACAGTACCC
AGCAGCTCACTTGGCACTCAGACCTATATCTGTAATGTCAACCATAAGCCATCTAACA
CTAAAGTCGACAAGAAGGTCGAGCCCAAGAGTTGCGACAAAACACATACCTGCCCC
CCATGTCCCGCACCAGAACTCCTGGGTGGTCCAAGCGTGTTTTTGTTTCCACCTAAAC
CAAAGGACACCCTGATGATTAGCCGTACACCAGAGGTGACTTGCGTGGTGGTCGACG
TGAGTCACGAGGATCCAGAGGTGAAGTTCAACTGGTATGTGGATGGCGTTGAAGTAC
ATAACGCCAAAACCAAGCCCAGAGAGGAACAATACAATAGCACCTACCGAGTCGTCA
GTGTGCTGACCGTGCTTCACCAAGACTGGCTCAATGGGAAAGAGTACAAGTGTAAGG
TTAGCAATAAAGCTCTGCCTGCCCCAATCGAGAAGACTATCTCTAAGGCAAAGGGAC
AGCCTAGGGAGCCACAGGTCTATACACTCCCCCCCTCACGGGATGAGTTGACTAAGA
ATCAGGTGTCACTGACATGCCTTGTTAAGGGGTTTTATCCCTCTGACATCGCCGTCGA
ATGGGAGAGTAACGGACAGCCAGAGAACAACTACAAGACAACCCCACCAGTCCTGG
ACTCTGACGGCTCCTTCTTTCTGTATTCAAAGCTGACTGTGGACAAATCTAGATGGCA
GCAGGGCAACGTCTTTAGTTGTAGTGTGATGCATGAGGCTCTGCATAACCATTATACA
CAGAAAAGTCTCAGTCTTAGCCCAGGAAAA (SEQ ID NO:121).

CAAGTTCAGTTGCAGCAACCTGGTGCAGAATTGGTGAGACCCGGTACAT
CTGTCAAGATGAGCTGCAAGGCCAGCGGGTACACCTTTACTAGTTACTGGATGCATTG
GGTGAAACAGCGACCCGGACAAGGATTGGAGTGGATCGGTGATATCTATCCAGGGTC
CGACAGCACAAACTATCATGAGAAGTTTAAGAGCAAGGCCACCCTGACAGTCGACA
CCTCAAGCTCCACAGCTTACATGCAGCTGTCTTCCTTGACTAGCGAAGATTCTGCCGT
GTACTATTGCGCTCTTGATAGCTCTGGGTATGGGTTCGCCTATTGGGGACAGGGTACTC
TGGTGACAGTGTCCGCTGCCTCCACCAAAGGTCCCAGTGTATTTCCCTTGGCTCCATC
TAGCAAGTCTACATCCGGCGGTACAGCCGCACTGGGGTGTCTTGTGAAGGACTACTT
CCCTGAGCCTGTTACAGTATCTTGGAATTCAGGCGCTCTGACCTCAGGAGTCCACACC
TTTCCCGCTGTCTTGCAGTCTAGCGGCCTTTATAGCCTGTCTTCCGTGGTGACTGTTCC
CTCTAGTAGTCTCGGAACCCAGACCTACATCTGCAATGTGAATCACAAGCCCAGCAA

CACAAAAGTCGACAAGAAGGTGGAGCCTAAATCTTGCGACAAGACTCACACCTGTC
CTCCATGCCCTGCTCCAGAACTGCTGGGGGACCTTCAGTGTTCTCTTCCCTCCAAA
ACCAAAGGACACCCTCATGATCTCCCGCACCCCAGAGGTCACCTGTGTGGTGGTGGA
CGTTTCACATGAGGATCCTGAGGTGAAGTTCAATTGGTATGTGGACGGTGTCGAAGTT
CATAATGCCAAGACAAAGCCTCGCGAAGAGCAGTACAACAGCACTTACAGGGTGGT
GTCCGTCCTGACTGTCTTGCACCAGGACTGGTTGAACGGCAAAGAATACAAATGCAA
GGTGAGTAACAAGGCACTGCCTGCACCAATTGAGAAGACTATCTCTAAGGCCAAAGG
CCAGCCTCGTGAGCCTCAAGTGTATACCCTCCCCCCTAGCAGAGACGAGTTGACAAA
GAATCAGGTCAGCTTGACATGCCTGGTGAAGGGATTTTATCCCTCAGACATTGCCGTC
GAATGGGAGAGTAACGGCCAGCCAGAAAATAACTACAAGACAACCCCCCCTGTCCT
GGACAGCGATGGATCATTTTTCCTGTACTCAAAGCTGACTGTAGACAAGTCTCGATGG
CAGCAAGGTAACGTATTCTCCTGCAGCGTGATGCACGAGGCACTGCATAATCACTATA
CCCAGAAGAGCCTGAGCCTGTCACCAGGGAAG (SEQ ID NO:122).

GAGGTACAGTTGGTTGAATCTGGCGGTGGACTTGTACAACCAGGCGAAT
CCTTGAAACTGAGTTGCGACTCTAATGACTATGAATTCCCTAGTCACGATATGTCTTGG
GTTCGGAAGACCCCTGAGAAACGGCTTGAGCTTGTGGCAGCCATCAACTCAGACGG
TGGAAATACATATTACCCCGACACTATGGAGAGGAGATTTATTATCAGCCGCGATAATA
CCAAAAGGACACTTTATCTCCAGATGTCCTCTTTGCGCAGCGAGGATACCGCTCTGCA
TTATTGTGCCCGCCCACGACCAACTATCGGCACAACAGCCACTGGGTCTTCTATGTCT
GGGGCACAGGGCCCAAGAAGCGCCTCTACAAAGGGCCCTTCAGTGTTTCCCCTTGCT
CCATCATCTAAAAGTACTAGTGGAGGCACTGCTGCTCTCGGCTGTCTGGTCAAGGACT
ACTTTCCAGAGCCTGTGACCGTGTCCTGGAATAGTGGGGCACTTACATCTGGTGTACA
CACCTTTCCTGCTGTACTGCAGAGCTCTGGCCTGTATTCCCTGAGCAGCGTTGTCACA
GTCCCATCAAGTAGCTTGGGAACTCAGACCTACATATGCAATGTGAATCACAAACCAT
CCAACACCAAGGTGGATAAGAAGGTCGAGCCTAAAAGTTGTGACAAAACCCATACAT
GCCCACCTTGTCCTGCTCCTGAGCTTCTGGGCGGCCCAAGTGTTTCCTTTTCCCTCC
CAAACCCAAAGACACACTCATGATTTCCCGTACACCCGAGGTTACTTGTGTGGTTGTT
GATGTCAGCCACGAGGATCCTGAGGTCAAGTTCAACTGGTATGTCGACGGCGTTGAA
GTCCACAACGCTAAGACCAAGCCTCGGGAGGAGCAGTATAATTCCACTTACAGGGTG
GTTTCCGTTTTGACCGTGCTGCACCAGGATTGGCTGAACGGCAAAGAGTATAAATGC
AAGGTAAGCAATAAAGCCTTGCCCGCACCCATCGAGAAAACTATTAGCAAAGCCAAG

GGTCAGCCCAGAGAACCTCAAGTTTATACCCTCCCTCCCTCACGCGATGAACTCACTA
AGAATCAGGTGTCACTCACATGTCTCGTGAAGGGTTTTACCCCTCCGATATTGCTGT
GGAGTGGGAGTCTAACGGCCAGCCCGAGAACAATTACAAGACCACCCCACCTGTGC
TTGATAGCGATGGCTCCTTTTTCCTCTACAGTAAGCTGACAGTGGACAAATCTCGGTG
GCAGCAGGGCAACGTTTTCTCCTGTTCAGTAATGCATGAGGCATTGCATAATCATTATA
CCCAAAAGAGCCTCTCACTTTCCCCAGGGAAG (SEQ ID NO:123).

TCTATTGTGATGACTCAAAGTCCTAAATTTCTCCTCGTTTCCGCTGGAGAC
AGGGTGATAATAACTTGCAAAGCTTCACAGAGCGTGTCCAATGATGTAGTATGGTACC
AGCAAAAGCCAGGCCAGAGCCCAAAACTTCTGATCTACTACGGTTCACACCGGAAC
ACCGGAGTACCTACAAGGTTTACTGGTTCCGGCTATGGGACTGACTTCACATTCACTA
TTTCAACCGTCCAGGCTGAAGACCTGGCAGTCTATTTTTGTCAGCAGGATTTCTTCTC
ACCATGGACCTTCGGTGGCGGTACAAAATTGGAGATAAAGCGCACTGTTGCCGCCCC
TTCCGTCTTTATATTTCCACCCTCCGATGAGCAGCTTAAGTCAGGGACAGCCTCTGTC
GTGTGTCTGCTCAACAATTTTTACCCCCGTGAAGCCAAGGTGCAGTGGAAAGTGGAT
AACGCCCTGCAGTCTGGCAATTCTCAGGAGTCAGTGACCGAACAGGATTCAAAAGA
CTCTACATATAGCTTGTCATCCACCCTTACTCTGTCCAAAGCAGATTATGAGAAGCACA
AAGTTTATGCTTGTGAAGTTACTCACCAGGGTCTGAGCAGCCCCGTAACAAAGAGTT
TCAACCGGGGGGAGTGC (SEQ ID NO:124).

GACATAGTTTTGACCCAGTCTCCTGCTAGTCTCGCCGTGTCTCTGGGACA
ACGAGCAACTATTAGCTGCCGAGCCAGCGAGAGCGTTGACGAATACGGATTTCTTT
CATGAACTGGTTCCAGCAGAAGCCAGGACAGCCCCCACGTCTGCTTATTTACAGCGC
CTCTAACCAAGGGAGTGGAGTGCCAGCCAGATTTACAGGTAGTGGATCTGGAACAGA
TTTCTCCCTCAACATTCTGCCCATGGAGGAAGACGACACCGCTATGTATTTCTGTCAG
CAGAGCAAAGAAGTGCCCTGGACCTTTGGGGGAGGGACCAAACTCGAAATTAAGCG
AACTGTGGCTGCTCCCTCAGTCTTTATCTTCCCACCATCTGATGAGCAACTGAAGTCT
GGCACTGCATCCGTGGTGTGTCTGCTGAATAATTTTTACCCTCGCGAGGCAAAAGTGC
AGTGGAAGGTGGATAATGCTTTGCAGTCAGGAAATTCTCAGGAGAGTGTTACCGAAC
AAGATAGCAAGGACTCCACATATAGTTTGTCCTCTACACTTACTCTCTCCAAGGCAGA
CTACGAAAAACACAAGGTGTACGCTTGTGAGGTGACCCACCAGGGCCTGAGCTCAC
CAGTGACCAAATCCTTCAACCGCGGCGAGTGC (SEQ ID NO:125).

AGTATTGTGATGACTCAGACCCCCAAGTTTCTTTTGGTGAGTGCAGGTGA

CAGAGTGACAATTACCTGCAAAGCTTCACAGAGCGTCAGCAACGATGTCGTCTGGTA
CCGACAGAAGCCAGGACAGAGCCCCAAGCTCCTCATCTACTACGCCTCTAATCGATA
CATCGGCGTGCCCGACAGGTTTACCGGTTCTGGCTATGGTACTGATTTACCTTCACAA
TTTCTACTGTTCAGGCCGAAGATCTTGCCGTGTACTTTTGTCAACAGGATTACTATAGT
CCCTGGACTTTCGGCGGGGGTACTAAGCTGGAGATTAAGCGAACAGTGGCCGCACCC
TCCGTGTTTATCTTCCCACCCTCTGACGAGCAACTCAAGAGTGGAACTGCCTCTGTCG
TGTGCTTGCTGAACAACTTTTATCCCAGAGAAGCCAAGGTGCAGTGGAAGGTGGATA
ATGCCCTGCAGAGCGGGAACTCTCAGGAATCCGTCACAGAACAGGATTCAAAGGAC
TCAACTTATTCCCTTTCTTCAACCCTGACACTCTCCAAGGCCGACTACGAGAAGCATA
AAGTGTACGCTTGCGAGGTAACCCATCAGGGTCTCAGTAGCCCCGTCACTAAGAGCT
TCAACAGGGGCGAATGC (SEQ ID NO:126).

GATGTGGTTATGACTCAGTCCCCATTGTCCTTGCCAGTCTCTCTGGGTGAT
CAGGCATCTATTTCCTGCAGGTCCAGCCAGTCACTGGTGCATTCTAACGGAACACCT
ACCTTCACTGGTACTTGCAAAAGCCCGGGCAGAGCCCTAAGCTGCTCATTTATAAGGT
TTCTAATAGATTCAGCGGTGTGCCTGACAGATTTTCTGGTTCTGGATCTGGCACCGATT
TTACCCTGAAGATCAGTCGAGTAGAGGCCGAAGACTTGGGAGTGTACTTTTGCTCCC
AATCCACCCATGTCCCACCAGATGTGCGATGGCGTCATCAGGCTGGCAACCAAAGGA
CTGTGGCTGCCCCTAGTGTTTTTATATTCCCACCTTCAGACGAGCAGCTTAAAAGTGG
CACCGCCTCAGTGGTTTGTTTGCTGAATAATTTCTATCCAAGGGAAGCAAAGGTTCAA
TGGAAGGTGGACAACGCTCTGCAAAGCGGAAACAGTCAGGAGAGCGTGACCGAAC
AGGATAGCAAGGACAGTACTTATAGTTTGAGCAGTACTCTCACTTTGTCCAAAGCCGA
CTACGAGAAGCACAAGGTGTACGCCTGTGAAGTGACCCATCAGGGGTTGTCAAGCCC
TGTAACTAAAAGTTTTAATAGAGGCGAGTGT (SEQ ID NO:127).

GATATCCAGATGACCCAGACCACTTCCAGTCTTTCTGCTAGTCTCGGCGA
TCGAGTGACCATCAGCTGCCGAGCATCTCAGGACATTTCAAATTCACTCAACTGGTAT
CAACAGAAACCAGATGGCACCGTCAAGCTGCTGATTTATTACACCAGCAGACTCCAC
AGTGGTGTGCCCAGCAGGTTCACAGGTTCTGGGAGTGGGACTGATTACTCCCTGACA
ATTTCTAACTTGGAACAGGAGGATATCGCCACTTATTTTTGCCAGCAGGGCAACACCC
TTCCATTGACATTCGGCGCAGGAACCAAGCTTGAGTTGAAGAGAACCGTCGCCGCAC
CCTCTGTATTTATATTCCCACCATCTGACGAGCAACTGAAGTCCGGAACAGCCAGTGT
GGTGTGCCTCCTTAATAATTTTTACCCTCGTGAAGCCAAGGTACAGTGGAAGGTTGAT

AACGCCCTGCAGAGTGGAAATAGTCAGGAGTCTGTAACCGAGCAGGATTCCAAGGAT
TCCACATATTCACTGTCCAGTACATTGACATTGTCAAAGGCTGACTACGAAAAGCATA
AGGTATACGCCTGCGAAGTGACACATCAGGGGCTGTCTTCTCCCGTGACAAAGTCAT
TCAATCGCGGGGAGTGT (SEQ ID NO:128).

GATATTCAGATGAACCAGTCTCCCTCCAGTTTGTCCGCCTCCCTCGGTGAT
ACTATTACTATTACATGCCACGCTAACCAAAATATCTACGTGTGGCTGTCATGGTATCA
ACAGAAACCTGGCAACATTCCCAAGCTTCTCATCTACGAGGCATCAAACCTGCACAC
TGGCGTCCCTTCCAGGTTCTCAGGCTCAGGTTCCGGAACCGGTTTACACTGACCATA
AGTAGCCTGCAGCCTGAAGACATCGCAACCTACTATTGTCAGCAAGGCCAGAGCTAC
CCCTACACCTTCGGCGGAGGAACAAAGTTGGAGATAAAACGGACTGTCGCCGCACCA
TCTGTCTTCATCTTCCCCCCCAGTGATGAGCAGCTTAAGAGTGGGACTGCTTCTGTCG
TCTGTCTGCTCAATAATTTTTATCCAAGAGAGGCTAAGGTACAGTGGAAAGTGGACAA
CGCTCTGCAGTCTGGTAACTCCCAGGAGAGTGTGACCGAACAGGATTCTAAGGACTC
CACATACTCCCTGAGCTCTACACTTACACTCAGTAAAGCAGACTATGAGAAGCATAAA
GTGTATGCATGTGAGGTGACACACCAGGGTCTGTCCTCTCCTGTGACCAAGTCTTTCA
ACAGAGGCGAGTGT (SEQ ID NO:129).

GATATCGTCATGTCACAGAGCCCATCTAGCTTGGCTGTCTCAGTGGGCGA
GAAAGTGACCATGTCATGCAAAAGCTCCCAATCCTTGTTGTACAGCCGCAATCAGAA
AAATCGCCTTGCCTGGTACCAGCAGAAACCAGGTCAGTCCCCAAAACTGCTGATTTA
TTGGGCCAGCACAAGGGAGAGTGGTGTGCCCGATAGATTCACTGGGTCCGGGTCAG
GCACCGATTTCACTCTGACCATCTCATCTGTTAAAGCAGAAGATCTGGCCGTCTACTA
TTGCCAGCAGTACTATAGTTATCCCAGAACTTTCGGGGGCGGCACCAACCTGGAGATT
AAGCGTACCGTGGCAGCACCATCTGTCTTTATTTTCCCCCCATCAGACGAGCAGTTGA
AATCAGGAACCGCCTCTGTCGTGTGCCTGCTGAACAATTTTTATCCAAGAGAGGCTAA
GGTGCAGTGGAAGGTCGATAATGCTCTTCAGAGCGGTAATTCTCAAGAATCCGTGAC
TGAACAGGATTCAAAAGATAGCACATACAGTCTCTCCAGCACCCTGACCCTCAGCAA
GGCCGACTACGAGAAGCACAAAGTGTATGCCTGTGAAGTGACACATCAAGGTCTCTC
CAGCCCTGTAACTAAGAGTTTTAATAGAGGAGAATGC (SEQ ID NO:130).

GATGTCCTGATGACCCAGACTCCATTGAGCCTTCCTGTCTCCTTGGGGA
TCAAGCCTCCATTTCATGCAGGAGCTCCCAGAGCATAGTCCACACAGATGGAAACAC
TTACCTGGAGTGGTACTTGCAGAAGCCAGGCCAGTCTCCCAAACTGCTCATCTACAA

AGTGTCTAATAGATTTTCCGGTGTGCCCGACCGTTTTCAGGTAGCGGCTCTGGTACT
GATTTCACTCTGAAGATCTCCCGGGTGGAGGCTGAGGATCTGGGCGTCTATTATTGTT
TCCAGGGCTCCCACGTGCCATGGACCTTCGGTGGTGGCACCAAGTTGGAGATTAAGA
GGACAGTGGCCGCACCATCCGTGTTCATATTCCCCCTAGCGATGAACAGTTGAAAAG
CGGTACTGCCTCCGTGGTTTGTCTGCTGAATAATTTCTACCCTAGAGAAGCTAAAGTG
CAATGGAAGGTTGACAATGCCTTGCAGTCAGGCAATTCTCAGGAGTCTGTGACCGAA
CAGGACAGCAAGGACTCCACCTATTCTCTGTCTTCAACACTGACACTTAGTAAGGCT
GATTACGAAAAGCATAAGGTATACGCCTGTGAGGTGACACACCAGGGCCTGAGTTCA
CCAGTGACCAAGTCCTTCAATCGCGGGGAGTGT (SEQ ID NO:131).

GATATTGTGCTTACACAGTCCCCAGCTTCCCTGGCCGTTTCCCTTGGGCA
GCGAGCCACAATCTCCTGCCGCATCTGAGTCCGTTGATAGCTACGGCAATTCTTTT
ATGCACTGGTACCAACAAAAACCTGGTCAGCCTCCAAAGTTGCTGATTTATCGCGCTT
CCAACCTTGAGTCCGGTATTCCTGCTCGCTTTAGCGGCTCTGGGAGTAGAACCGACTT
CACACTGACCATTAACCCAGTTGAGGCTGATGATGTGGCTACCTATTACTGTCAGCAG
TCTAACGAAGACCCATTTACATTTGGGTCAGGCACTAAGCTGGAGATTAAGCGTACCG
TGGCCGCCCCAAGCGTTTTCATCTTCCCCCCAAGCGACGAGCAGCTGAAGTCCGGAA
CTGCCTCCGTCGTTTGTCTCTTGAACAATTTTTATCCTAGGGAAGCAAAAGTACAGTG
GAAGGTAGATAACGCTCTGCAAAGCGGTAACTCCCAAGAATCCGTAACCGAACAAGA
CTCAAAGGACAGTACTTATTCCCTGTCCAGCACCCTGACTCTTTCTAAAGCTGATTAT
GAAAAGCATAAAGTTTATGCATGTGAGGTGACCCACCAGGGATTGTCCTCCCCAGTTA
CTAAATCTTTTAACCGGGGGGAATGC (SEQ ID NO:132).

AATATCGTCATGACCCAGACTCCTAAATTTCTTCTGGTCTCCGCTGGAGA
CCGCGTCACTATAACTCGTAAAGCCAGCCAGTCCGTGTCTAATGACGTTGTGGTAC
CAGCAGAAGCCCGGGCAGTCCCCTAAGCTGCTCATGTATTACGCAAGTTATCGATACA
CCGGCGTGCCCGATCGATTCACCGGATCCGGTTATGGCACCGACTTCACATTTACTATT
TCTAACGTTCAGGCCGAGGACCTCGCCGTATACTTTTGTCAACAAGACTACTCCAGCC
CTTTTACATTTGGAAGCGGGACAAAATTGGAGATCAAGAGGACTGTTGCCGCACCTT
CCGTGTTTATATTTCCACCTTCCGACGAGCAGCTGAAGTCCGGGACCGCAAGCGTGG
TTTGTCTCCTCAATAATTTCTATCCTCGAGAGGCTAAGGTCCAGTGGAAGGTGGACAA
TGCTCTGCAGAGTGGCAACTCTCAGGAGTCAGTTACTGAACAAGACAGCAAGGACA
GCACATATTCACTGTCATCCACTCTTACTCTCTCCAAGGCTGACTACGAAAAGCATAA

GGTTTATGCCTGCGAGGTTACTCATCAGGGTCTGTCCAGTCCAGTGACCAAATCTTTT
AATAGAGGAGAGTGC (SEQ ID NO:133).

GATATTGTCTTGACCCAATCACCTGCTAGCCTGGCTGTGAGCTTGGGGCA
GCGAGCAACCATCTCCTGTAGGGCCTCTCAGTCCGTCTCCACAAGTACTTACAACTAC
ATGCATTGGTACCAGCAGAAACCTGGCCAACCACCAAAATTGCTCATTAAGTATGCTA
GCAACCTTGAGTCAGGCGTCCCCGCAAGGTTTAGCGGATCTGGCTCAGGAACCGATT
TTACTCTGAACATTCACCCCGTGGAGGAGGAGGACACTGCTACCTACTATTGCCAGCA
TAGTTGGGAGATACCCTTCACCTTTGGCAGTGGCACAAAGCTCGAGATTAAGCGCAC
TGTTGCCGCACCCAGCGTGTTTATCTTTCCACCTAGCGACGAACAGCTTAAGTCTGGT
ACTGCTAGTGTGGTGTGTTTGTTGAATAACTTCTACCCCCGTGAAGCCAAGGTTCAGT
GGAAAGTGGATAACGCTCTCCAGAGCGGGAACAGTCAGGAGTCTGTTACCGAACAG
GACTCAAAGGACAGCACTTACAGCCTGAGCAGTACTCTGACCCTGAGTAAGGCCGAT
TATGAAAAGCACAAGGTGTACGCCTGTGAGGTGACCCACCAGGGTCTGTCATCCCCT
GTCACCAAGTCTTTTAATCGGGGTGAATGC (SEQ ID NO:134).

Wherein, in the present application, the nucleotide sequences shown in the above SEQ ID NO: 113 and 124 respectively encode the heavy chain and light chain of 3-6A4 antibody, the nucleotide sequences shown in the above SEQ ID NO: 114 and 125 respectively encode the heavy chain and light chain of 5-20E11-5F2 antibody, the nucleotide sequences shown in the above SEQ ID NO: 115 and 126 respectively encode the heavy chain and light chain of 3-5B2-2G4 antibody, the nucleotide sequences shown in the above SEQ ID NO: 116 and 127 respectively encode the heavy chain and light chain of 3-5B2-7A4 antibody, the nucleotide sequences shown in the above SEQ ID NO: 117 and 128 respectively encode the heavy chain and light chain of 3-2B5-3D2 antibody, the nucleotide sequences shown in the above SEQ ID NO: 118 and SEQ ID NO:129 respectively encode the heavy chain and light chain of 3-7C9-1A6 antibody, the nucleotide sequences shown in the above SEQ ID NO: 119 and SEQ ID NO:130 respectively encode the heavy chain and light chain of 3-9E1-3A2 antibody, the nucleotide sequences shown in the above SEQ ID NO: 120 and SEQ ID NO:131 respectively encode the heavy chain and light chain of 3-15D5-3A6 antibody, the nucleotide sequences shown in the above SEQ ID NO: 121 and SEQ ID NO:132 respectively encode the heavy chain and light chain of 3-15D5-6A1 antibody, the nucleotide sequences shown in the above SEQ ID NO: 122 and SEQ ID NO:133 respectively encode the heavy chain and light chain of 5-20E11-1A2 antibody, the nucleotide sequences shown in the above SEQ ID NO: 123 and SEQ ID NO: 134 respectively encode the heavy chain and light chain of 5-21G2-1D3 antibody.

According to the embodiments of the present invention, the nucleic acid molecule has a nucleotide sequence shown in any one of SEQ ID NO: 159-162 or has a nucleotide sequence shown in any one of SEQ ID NO: 163-166.

CAAGTGCAGCTGGTTCAGAGCGGAGCTGAGGTGAAGAAGCCTGGAGCT
AGCGTGAAGGTGTCTTGTAAAGCCAGCGACTACACCTTTACCAATTACTGGCTGGGC
TGGGTCAGACAGGCCCCTGGCCAGGGCCTGGAATGGATGGGCCACATCTACCCCGGC
GGAGGCTACACAAACTACAACGAGAAGTTCAAGGGCAGAGTGACAATGACCAGAGA
TACAAGCACCAGCACAGTGTATATGGAACTGTCTAGCCTGCGGAGCGAGGACACCGC
CGTGTACTACTGCGCCAGAGTGCGGTCCGGCAACGACGCCCTGGATTTCTGGGGCCA
GGGCACCCTGGTGACCGTGTCCAGCGCCAGCACCAAGGGCCCTTCCGTGTTTCCACT
GGCCCCCTCCTCTAAATCCACATCTGGCGGCACCGCCGCCCTGGGCTGTCTGGTGAA
GGACTACTTCCCAGAGCCTGTGACAGTGTCCTGGAACTCTGGCGCCCTGACATCCGG
CGTGCACACATTTCCAGCCGTGCTGCAGAGCTCCGGCCTGTACAGCCTGTCTAGCGT
GGTGACAGTGCCCTCCTCTAGCCTGGGCACACAGACCTATATCTGCAACGTGAATCAC
AAGCCAAGCAATACCAAGGTGGACAAGAAGGTGGAGCCCAAGTCCTGTGATAAGAC
ACACACCTGCCCCCCTTGTCCTGCTCCCGAGCTGCTGGGCGGCCCTAGCGTGTTCCT
GTTTCCACCCAAGCCTAAGGACACCCTGATGATCTCCCGGACACCCGAGGTGACCTG
CGTGGTGGTGGACGTGTCTCACGAGGATCCTGAGGTGAAGTTCAACTGGTATGTGGA
TGGCGTGGAGGTGCACAATGCCAAGACCAAGCCCAGAGAGGAGCAGTACAACTCTA
CATATAGGGTGGTGAGCGTGCTGACCGTGCTGCACCAGGACTGGCTGAACGGCAAGG
AGTATAAGTGCAAGGTGTCCAATAAGGCCCTGCCCGCCCCCATCGAGAAGACAATCA
GCAAGGCCAAGGGCCAGCCTCGGGAGCCACAGGTGTACACCCTGCCTCCATCCAGA
GACGAGCTGACAAAGAACCAGGTGTCTCTGACATGTCTGGTGAAGGGCTTCTATCCT
AGCGATATCGCCGTGGAGTGGGAGTCCAATGGCCAGCCAGAGAACAATTACAAGACC
ACACCCCCTGTGCTGGACTCCGATGGCTCCTTCTTTCTGTATTCCAAGCTGACCGTGG
ATAAGTCTCGGTGGCAGCAGGGCAACGTGTTCAGCTGTTCCGTGATGCACGAAGCCC
TGCATAATCACTATACTCAGAAATCCCTGTCCCTGTCACCTGGAAAGTGATAA (SEQ ID
NO:159).

CAAGTGCAGCTGGTCCAGAGCGGCGCCGAGGTGAAAAAGCCTGGAGCT
AGCGTGAAGGTGTCCTGCAAGGCCAGCGACTACACCTTCACCAACTATTGGCTGGGC
TGGGTGCGGCAGGCCCCTGGACAGGGCCTGGAATGGATCGGCCACATCTACCCCGGA
GGCGGCTACACCAATTACAACGAGAAGTTCAAGGGCAGAGTGACCATGACCGCCGAT
ACAAGCACATCTACAGTGTACATGGAACTGAGCAGCCTGAGAAGCGAGGATACAGCT
GTTTACTTCTGTGCCAGAGTGCGGTCCGGCAACGACGCCCTGGACTTTTGGGGCCAG
GGCACCCTGGTGACCGTGTCTAGCGCCAGCACCAAGGGCCCTTCCGTGTTTCCACTG
GCCCCCTCCTCTAAATCCACATCTGGCGGCACCGCCGCCCTGGGCTGTCTGGTGAAG
GACTACTTCCCAGAGCCTGTGACAGTGTCCTGGAACTCTGGCGCCCTGACATCCGGC
GTGCACACATTTCCAGCCGTGCTGCAGAGCTCCGGCCTGTACAGCCTGTCTAGCGTG
GTGACAGTGCCCTCCTCTAGCCTGGGCACACAGACCTATATCTGCAACGTGAATCACA
AGCCAAGCAATACCAAGGTGGACAAGAAGGTGGAGCCCAAGTCCTGTGATAAGACA
CACACCTGCCCCCCTTGTCCTGCTCCCGAGCTGCTGGGCGGCCCTAGCGTGTTCCTGT
TTCCACCCAAGCCTAAGGACACCCTGATGATCTCCCGGACACCCGAGGTGACCTGCG
TGGTGGTGGACGTGTCTCACGAGGATCCTGAGGTGAAGTTCAACTGGTATGTGGATG
GCGTGGAGGTGCACAATGCCAAGACCAAGCCCAGAGAGGAGCAGTACAACTCTACA
TATAGGGTGGTGAGCGTGCTGACCGTGCTGCACCAGGACTGGCTGAACGGCAAGGA
GTATAAGTGCAAGGTGTCCAATAAGGCCCTGCCCGCCCCCATCGAGAAGACAATCAG
CAAGGCCAAGGGCCAGCCTCGGGAGCCACAGGTGTACACCCTGCCTCCATCCAGAG
ACGAGCTGACAAAGAACCAGGTGTCTCTGACATGTCTGGTGAAGGGCTTCTATCCTA
GCGATATCGCCGTGGAGTGGGAGTCCAATGGCCAGCCAGAGAACAATTACAAGACCA
CACCCCCTGTGCTGGACTCCGATGGCTCCTTCTTTCTGTATTCCAAGCTGACCGTGGA
TAAGTCTCGGTGGCAGCAGGGCAACGTGTTCAGCTGTTCCGTGATGCACGAAGCCCT
GCATAATCACTATACTCAGAAATCCCTGTCCCTGTCACCTGGAAAGTGATAA (SEQ ID
NO:160).

CAAGTGCAGCTGGTCCAGAGCGGCGCCGAGGTGAAAAAGCCTGGCGCT
TCTGTGAAGGTGTCCTGCAAGGCCAGCGACTACACATTTACCAATTATTGGCTGGGCT
GGGTGCGGCAGGCCCCTGGACAGGGCCTGGAATGGATCGGCCACATCTACCCCGGCG
GAGGCTACACAAACTACAACGAGAAGTTCAAGGGCAGAGCCACACTCACCGCTGAT
ACCAGCACAAGCACCGTGTACATGGAACTGAGCAGCCTGAGAAGCGAGGACACCGC
CGTGTACTTCTGTGCCAGAGTGCGGTCCGGCAACGACGCCCTGGATTTCTGGGGACA

GGGCACCCTGGTGACCGTGTCTAGCGCCAGCACCAAGGGCCCTTCCGTGTTTCCACT
GGCCCCCTCCTCTAAATCCACATCTGGCGGCACCGCCGCCCTGGGCTGTCTGGTGAA
GGACTACTTCCCAGAGCCTGTGACAGTGTCCTGGAACTCTGGCGCCCTGACATCCGG
CGTGCACACATTTCCAGCCGTGCTGCAGAGCTCCGGCCTGTACAGCCTGTCTAGCGT
GGTGACAGTGCCCTCCTCTAGCCTGGGCACACAGACCTATATCTGCAACGTGAATCAC
AAGCCAAGCAATACCAAGGTGGACAAGAAGGTGGAGCCCAAGTCCTGTGATAAGAC
ACACACCTGCCCCCCTTGTCCTGCTCCCGAGCTGCTGGGCGGCCCTAGCGTGTTCCT
GTTTCCACCCAAGCCTAAGGACACCCTGATGATCTCCCGGACACCCGAGGTGACCTG
CGTGGTGGTGGACGTGTCTCACGAGGATCCTGAGGTGAAGTTCAACTGGTATGTGGA
TGGCGTGGAGGTGCACAATGCCAAGACCAAGCCCAGAGAGGAGCAGTACAACTCTA
CATATAGGGTGGTGAGCGTGCTGACCGTGCTGCACCAGGACTGGCTGAACGGCAAGG
AGTATAAGTGCAAGGTGTCCAATAAGGCCCTGCCCGCCCCCATCGAGAAGACAATCA
GCAAGGCCAAGGGCCAGCCTCGGGAGCCACAGGTGTACACCCTGCCTCCATCCAGA
GACGAGCTGACAAAGAACCAGGTGTCTCTGACATGTCTGGTGAAGGGCTTCTATCCT
AGCGATATCGCCGTGGAGTGGGAGTCCAATGGCCAGCCAGAGAACAATTACAAGACC
ACACCCCCTGTGCTGGACTCCGATGGCTCCTTCTTTCTGTATTCCAAGCTGACCGTGG
ATAAGTCTCGGTGGCAGCAGGGCAACGTGTTCAGCTGTTCCGTGATGCACGAAGCCC
TGCATAATCACTATACTCAGAAATCCCTGTCCCTGTCACCTGGAAAGTGATAA (SEQ ID
NO:161).

CAAGTGCAGCTGGTTCAGAGCGGCGCCGAGGTGAAGAAGCCTGGCGCT
AGCGTGAAGGTGTCTTGTAAAGCCAGCGACTACACATTTACCAATTACTGGCTGGGAT
GGGTCCGGCAGGCCCCTGGCCACGGCCTGGAATGGATCGGCCACATCTACCCCGGCG
GAGGATATACAAACTACAACGAGAAGTTCAAGGGCAGAGCCACCCTGACCGCTGATA
CATCTACAAGCACCGTGTACATGGAACTGAGCAGCCTGAGAAGCGAGGATACCGCCG
TGTACTTCTGCGCCAGAGTGCGGTCCGGCAACGACGCCCTGGACTTCTGGGGCCAGG
GCACCCTGGTGACCGTGTCCAGCGCCAGCACCAAGGGCCCTTCCGTGTTTCCACTGG
CCCCCTCCTCTAAATCCACATCTGGCGGCACCGCCGCCCTGGGCTGTCTGGTGAAGG
ACTACTTCCCAGAGCCTGTGACAGTGTCCTGGAACTCTGGCGCCCTGACATCCGGCG
TGCACACATTTCCAGCCGTGCTGCAGAGCTCCGGCCTGTACAGCCTGTCTAGCGTGG
TGACAGTGCCCTCCTCTAGCCTGGGCACACAGACCTATATCTGCAACGTGAATCACAA
GCCAAGCAATACCAAGGTGGACAAGAAGGTGGAGCCCAAGTCCTGTGATAAGACAC

ACACCTGCCCCCCTTGTCCTGCTCCCGAGCTGCTGGGCGGCCCTAGCGTGTTCCTGTT
TCCACCCAAGCCTAAGGACACCCTGATGATCTCCCGGACACCCGAGGTGACCTGCGT
GGTGGTGGACGTGTCTCACGAGGATCCTGAGGTGAAGTTCAACTGGTATGTGGATGG
CGTGGAGGTGCACAATGCCAAGACCAAGCCCAGAGAGGAGCAGTACAACTCTACAT
ATAGGGTGGTGAGCGTGCTGACCGTGCTGCACCAGGACTGGCTGAACGGCAAGGAG
TATAAGTGCAAGGTGTCCAATAAGGCCCTGCCCGCCCCCATCGAGAAGACAATCAGC
AAGGCCAAGGGCCAGCCTCGGGAGCCACAGGTGTACACCCTGCCTCCATCCAGAGA
CGAGCTGACAAAGAACCAGGTGTCTCTGACATGTCTGGTGAAGGGCTTCTATCCTAG
CGATATCGCCGTGGAGTGGGAGTCCAATGGCCAGCCAGAGAACAATTACAAGACCAC
ACCCCCTGTGCTGGACTCCGATGGCTCCTTCTTTCTGTATTCCAAGCTGACCGTGGAT
AAGTCTCGGTGGCAGCAGGGCAACGTGTTCAGCTGTTCCGTGATGCACGAAGCCCTG
CATAATCACTATACTCAGAAATCCCTGTCCCTGTCACCTGGAAAGTGATAA (SEQ ID
NO:162).

GAGATCGTGCTGACACAGAGCCCTGCTACACTCTCCCTGTCCCCAGGCG
AGCGGGCCACACTGTCTTGTAGAGCCAGCGAGAGCGTCGACGAGTACGGCATTAGCT
TCATGAACTGGTATCAGCAGAAACCTGGCCAGGCCCCTAGACTGCTGATCTACAGCG
CCTCCAACCAGGGCAGCGGCATCCCCGCTAGATTCAGCGGCAGCGGATCTGGCACCG
ACTTCACCCTGACCATCAGCAGCCTGGAACCCGAGGATTTTGCCGTGTACTACTGCCA
GCAATCTAAGGAAGTGCCTTGGACCTTCGGCGGAGGCACCAAGCTGGAAATCAAGA
GGACAGTGGCCGCCCCAAGCGTGTTCATCTTTCCCCCTTCCGACGAGCAGCTGAAGT
CTGGCACCGCCAGCGTGGTGTGCCTGCTGAACAACTTCTACCCTCGGGAGGCCAAGG
TCCAGTGGAAGGTGGATAACGCCCTGCAGTCTGGCAATAGCCAGGAGTCCGTGACCG
AGCAGGACTCTAAGGATAGCACATATTCCCTGTCTAGCACCCTGACACTGAGCAAGG
CCGATTACGAGAAGCACAAGGTGTATGCCTGTGAAGTCACCCATCAGGGGCTGTCAT
CACCCGTCACTAAGTCATTCAATCGCGGAGAATGCTGATAA (SEQ ID NO:163).

GAGATCGTGCTGACCCAGAGCCCCGCCACCCTGTCCCTGAGCCCTGGCG
AGCGGGCTACACTGTCTTGTAGAGCCAGCGAGAGCGTCGACGAGTACGGCATCTCTT
TTATGAACTGGTATCAGCAGAAACCTGGACAGGCCCCTAGACTGCTGATCTACAGCG
CCTCCAACCAGGGCAGCGGCATTCCTGCTAGATTCAGCGGCAGCGGCTCCGGCACAG
ATTTCACCCTCACCATCAGCAGCCTGGAACCCGAGGACTTCGCCGTGTACTTCTGCCA
GCAATCTAAGGAAGTGCCATGGACATTCGGCGGAGGCACCAAGCTGGAAATCAAGA

GGACAGTGGCCGCCCCAAGCGTGTTCATCTTTCCCCCTTCCGACGAGCAGCTGAAGT
CTGGCACCGCCAGCGTGGTGTGCCTGCTGAACAACTTCTACCCTCGGGAGGCCAAGG
TCCAGTGGAAGGTGGATAACGCCCTGCAGTCTGGCAATAGCCAGGAGTCCGTGACCG
AGCAGGACTCTAAGGATAGCACATATTCCCTGTCTAGCACCCTGACACTGAGCAAGG
CCGATTACGAGAAGCACAAGGTGTATGCCTGTGAAGTCACCCATCAGGGGCTGTCAT
CACCCGTCACTAAGTCATTCAATCGCGGAGAATGCTGATAA (SEQ ID NO:164).

GAGATCGTGCTGACCCAGTCTCCAGCCACCCTCTCCCTGAGCCCCGGCG
AGCGGGCCACACTGTCTTGTAGAGCTAGCGAGAGCGTCGACGAGTACGGCATCTCCT
TCATGAACTGGTTCCAGCAGAAACCTGGCCAGGCCCCTAGACTGCTGATCTACAGCG
CCAGCAACCAGGGCTCCGGAATTCCTGCTAGATTCAGCGGCTCTGGCAGCGGCACAG
ATTTTACCCTGACAATCAGCAGCCTGGAACCCGAGGACTTTGCCGTGTACTTCTGCCA
GCAAAGCAAGGAAGTGCCTTGGACCTTCGGCGGAGGCACCAAGCTGGAAATCAAGA
GGACAGTGGCCGCCCCAAGCGTGTTCATCTTTCCCCCTTCCGACGAGCAGCTGAAGT
CTGGCACCGCCAGCGTGGTGTGCCTGCTGAACAACTTCTACCCTCGGGAGGCCAAGG
TCCAGTGGAAGGTGGATAACGCCCTGCAGTCTGGCAATAGCCAGGAGTCCGTGACCG
AGCAGGACTCTAAGGATAGCACATATTCCCTGTCTAGCACCCTGACACTGAGCAAGG
CCGATTACGAGAAGCACAAGGTGTATGCCTGTGAAGTCACCCATCAGGGGCTGTCAT
CACCCGTCACTAAGTCATTCAATCGCGGAGAATGCTGATAA (SEQ ID NO:165).

GAGATCGTGCTGACACAGAGCCCTGCTACACTCTCCCTGAGCCCCGGCG
AGCGGGCCACCCTGTCTTGTAGAGCCAGCGAGAGCGTGGACGAGTACGGCATTAGCT
TCATGAACTGGTTTCAGCAGAAACCTGGCCAGGCCCCTAGACTGCTGATCTACAGCG
CCTCCAACCAGGGCTCTGGCATCCCCGCTAGATTCAGCGGATCTGGCTCCGGCACCG
ACTTCACCCTGACCATCAGCAGCCTGGAACCAGAGGATTTTGCCATGTACTTCTGCCA
GCAAAGCAAGGAAGTGCCTTGGACCTTCGGCGGAGGCACAAAGCTGGAAATCAAGA
GGACAGTGGCCGCCCCAAGCGTGTTCATCTTTCCCCCTTCCGACGAGCAGCTGAAGT
CTGGCACCGCCAGCGTGGTGTGCCTGCTGAACAACTTCTACCCTCGGGAGGCCAAGG
TCCAGTGGAAGGTGGATAACGCCCTGCAGTCTGGCAATAGCCAGGAGTCCGTGACCG
AGCAGGACTCTAAGGATAGCACATATTCCCTGTCTAGCACCCTGACACTGAGCAAGG
CCGATTACGAGAAGCACAAGGTGTATGCCTGTGAAGTCACCCATCAGGGGCTGTCAT
CACCCGTCACTAAGTCATTCAATCGCGGAGAATGCTGATAA (SEQ ID NO:166).

In the third aspect of the present invention, the present invention provides an expression vector. According to the embodiments of the present invention, the expression vector carries the aforementioned nucleic acid molecule. After the expression vector according to the embodiments of the present invention is introduced into a suitable recipient cell, the expression of the aforementioned antibody or antigen-binding fragment thereof that specifically recognizes IL-11 can be effectively realized under the mediation of the regulatory system, thereby in vitro mass acquisition of the antibody or antigen-binding fragment are achieved.

According to the embodiments of the present invention, the above-mentioned expression vector may further include at least one of the following additional technical features:
According to the embodiments of the present invention, the expression vector is a eukaryotic expression vector. Thereby the aforementioned antibody or antigen-binding fragment thereof that specifically recognizes IL-11 can be expressed in eukaryotic cells, such as CHO cells.

In the fourth aspect of the present invention, the present invention provides a recombinant cell. According to the embodiments of the present invention, the recombinant cell carries the aforementioned nucleic acid molecule, or expresses the aforementioned antibody or antigen-binding fragment thereof. The recombinant cell according to the embodiments of the present invention can be used for the in vitro expression and mass acquisition of the aforementioned antibody or antigen-binding fragment thereof that specifically recognizes IL-11.

According to the embodiments of the present invention, the above-mentioned recombinant cell may further include at least one of the following additional technical features:
According to the embodiments of the present invention, the recombinant cell is obtained by introducing the aforementioned expression vector into a host cell.

According to the embodiments of the present invention, the expression vector is introduced into the host cell by electrotransduction.

According to the embodiments of the present invention, the recombinant cell is a eukaryotic cell.

According to the embodiments of the present invention, the recombinant cell is a mammalian cell.

In the fifth aspect of the present invention, the present invention provides a pharmaceutical composition. According to the embodiments of the present invention, the pharmaceutical composition comprises the aforementioned antibody, the aforementioned nucleic acid molecule, the aforementioned expression vector or the aforementioned recombinant cell. The antibody contained or expressed in the pharmaceutical composition according to the embodiments of the present invention can specifically target and bind to IL-11, and block the binding of IL-11 to the IL-11 receptor.

In the sixth aspect of the present invention, the present invention provides use of the aforementioned antibody, the aforementioned nucleic acid molecule, the aforementioned expression vector or the aforementioned recombinant cell, and the aforementioned pharmaceutical composition in the manufacture of a medicament, the medicament is used for treating pulmonary fibrosis, non-alcoholic steatohepatitis (NASH), chronic heart failure (CHF) or diseases caused by the transformation of fibroblasts into fibrosis.

In the seventh aspect of the present invention, the present invention provides a kit for detecting IL-11. According to the embodiments of the present invention, the kit includes any one of the aforementioned antibodies. The aforementioned IL-11 antibody can specifically target and bind to IL-11. The kit according to the embodiments of the present invention can realize the specific detection of IL-11. For example, when the antibody is bound with a fluorophore, a fluorescence detection device can be used to achieve localization or real-time detection of IL-11.

In the eighth aspect of the present invention, the present invention provides use of the aforementioned antibody, the aforementioned nucleic acid molecule, the aforementioned expression vector or the aforementioned recombinant cell in the preparation of a kit, the kit is used for detecting IL-11 or diagnosing IL-11-related diseases.

Additional aspects and advantages of the present invention will be set forth in part from the following description, and in part will be apparent from the following description, or may be learned by practice of the invention.

### Description of the drawings

The above and/or additional aspects and advantages of the present invention will become apparent and readily understood from the following description of embodiments taken in conjunction with the accompanying drawings, wherein:
Figure 1 is the result of competitive blocking the binding of IL-11 and IL-11R by a preferred antibody according to the embodiments of the present invention;
Figure 2 is the test result of competitive blocking activity of the preferred antibody according to the embodiments of the present invention;
Figure 3 and Figure 4 are the western test results of the preferred antibodies inhibiting the transformation of lung fibroblast HFL-1 to fibrosis according to the embodiments of the present invention;
Figure 5 is the statistical analysis result of the preferred antibodies inhibiting the transformation of lung fibroblasts HFL-1 to fibrosis according to the embodiments of the present invention;
Figure 6 and Figure 7 are results of inhibition of pulmonary fibrosis in C57BL/6 mice by the preferred antibody according to the embodiments of the present invention;
Figures 8, 9, 10 and 11 are results of inhibition of pulmonary fibrosis in Sprague Dawley rats by the preferred antibody according to the embodiments of the present invention; and
Figure 12 is the competitive blocking activity of the humanized antibody according to the embodiments of the present invention.

### EXAMPLES

The embodiments of the present invention are described in detail below, examples of the embodiments are illustrated in the accompanying drawings. The embodiments described below with reference to the accompanying drawings are exemplary, and are intended to explain the present invention and should not be construed as limiting the present invention.

In addition, the terms "first" and "second" are only used for descriptive purposes, and should not be construed as indicating or implying relative importance or implying the number of indicated technical features. Thus, a feature delimited with "first", "second" may expressly or implicitly include at least one of that feature. In the description of the present invention, "plurality" means at least two, such as two, three, etc., unless otherwise expressly and specifically defined.

### Antibody

As used herein, the term "antibody" is an immunoglobulin molecule capable of binding to a specific antigen. It consists of two light chains with lighter molecular weight and two heavy chains with heavier molecular weight. The heavy chain (H chain) and the light chain (L chain) are connected by a disulfide bond to form a tetrapeptide chain molecule. Among them, the amino acid sequence of the amino-terminal (N-terminus) of the peptide chain varies greatly, which is called variable region (V region), and the carboxyl-terminal (C-terminus) is relatively stable and varies little, which is called constant region (C region). The V regions of the L chain and H chain are called VL and VH, respectively.

The amino acid composition and arrangement sequence of certain regions in the variable region have a higher degree of variation, which is called hypervariable region (HVR). The hypervariable region is where the antigen and antibody bind, so it is also called complementarity-determining region (CDR). There are three CDR regions on both the heavy chain variable region and the light chain variable region. The amino acid composition and arrangement sequence outside the hypervariable region of the antibody variable region vary relatively little, which is called framework region (FR). There are four framework regions in VH and VL respectively, which are represented by FR1, FR2, FR3 and FR4, respectively.

The present invention uses an IL-11 antigen to obtain a highly specific and high-affinity anti-IL-11 Fab (antigen-binding fragment) antibody fragment through immunization. The antibody fragment can specifically bind to the IL-11 antigen, so that diseases such as pulmonary fibrosis can be targeted for treatment.

It should be noted that "immunogenicity" refers to the property that can cause an immune response, that is, an antigen can stimulate specific immune cells, to make the immune cells activate, proliferate, and differentiate, and finally produce the characteristics of immune effector antibodies and sensitized lymphocytes. The inventors of the present application further carried out humanization transformation (humanization modification) on the screened new neutralizing antibody sequences of murine origin to obtain a humanized antibody, which can not only retain the affinity and specificity of the parental mouse monoclonal antibody, but also greatly reduce its immunogenicity and improve its safety.

In some embodiments, the present invention provides an antibody or antigen-binding fragment that can specifically recognize IL-11, the antibody comprises CDRs selected from at least one of the following or an amino acid sequence having at least 95% identity thereto: a heavy chain variable region comprising CDRs having the amino acid sequence of SEQ ID NO: 1-33, a light chain variable region comprising CDRs having the amino acid sequence of SEQ IN NO: 34-66. In other embodiments, the antibody or antigen-binding fragment provided by the present invention has conservative amino acid substitution compared to the above heavy chain and light chain. An "antigen-binding fragment" refers to an antibody fragment that retains the ability to specifically bind an antigen. "Conservative amino acid substitution" refers to the replacement of an amino acid by another amino acid with a residue that is biologically, chemically, or structurally similar. Biologically similar means that the substitution does not destroy the biological activity of the IL-11 antibody or binding to the IL-11 antigen. Structurally similar means that amino acids have side chains of similar length, such as alanine, glycine, or serine, or have side chains of similar size. Chemically similar means that amino acids have the same charge or are both hydrophilic or hydrophobic. For example, the hydrophobic residues isoleucine, valine, leucine or methionine are substituted for each other. Alternatively, polar amino acids can be substituted for each other, e.g., arginine substituted for lysine, glutamate substituted for aspartic acid, glutamine substituted for asparagine, serine substituted for threonine, and the like.

In some embodiments, the present invention provides an antibody or antigen-binding fragment, the heavy chain variable region has the amino acid sequence shown in any one of SEQ ID NO: 67-77 and the light chain variable region has the amino acid sequence shown in any one of SEQ ID NO: 78-88. The inventors can obtain the CDR regions of the above-mentioned heavy chain variable region (as shown in SEQ ID NO: 1-33) and the CDR regions of the light chain variable region (as shown in SEQ ID NO: 34-66) through the antibody sequence alignment database (NCBI, IMGT). In other embodiments, the heavy chain variable region of the antibody or antigen-binding fragment have conservative amino acid substitution compared to the amino acid sequence shown in SEQ ID NO: 67-77. In other embodiments, the light chain variable region of the antibody or antigen-binding fragment have conservative amino acid substitution compared to the amino acid sequence shown in any one of SEQ ID NO: 78-88. Of course, these conservative amino acid substitutions do not alter the biological function of the antibody or antigen-binding fragment. In some embodiments, these conservative amino acid substitutions can occur at the amino acids in heavy and light chain variable regions other than the CDR regions.

In some preferred embodiments, the present invention provides an anti-IL-11 antibody, the heavy chain has the amino acid sequence shown in any one of SEQ ID NO: 91-101 and the light chain has the amino acid sequence shown in any one of SEQ ID NO: 102-112.

In some preferred embodiments, the present invention provides an anti-IL-11 single chain antibody.

### Nucleic acid molecule, expression vector, recombinant cell

In the process of preparing or obtaining these antibodies, the nucleic acid molecules expressing these antibodies can be used to connect with different vectors, and then express in different cells to obtain corresponding antibodies.

To this end, the present invention also provides an isolated nucleic acid molecule, which encodes the antibody or antigen-binding fragment described above.

In some embodiments, the isolated nucleic acid molecule has the nucleotide sequence shown in any one of SEQ ID NO: 113-123 or has the nucleotide sequence shown in any one of SEQ ID NO: 124-134.

In some embodiments, the isolated nucleic acid molecule has at least more than 90% homology with the nucleotide sequences shown in SEQ ID NO: 113-123 above, preferably, more than 95% homology, more preferably, more than 98% or 99% homology. In at least some embodiments, the isolated polynucleotide has at least more than 90% homology with the nucleotide sequences shown in SEQ ID NO: 124-134, preferably, more than 95% homology, more preferably, more than 98% or 99% homology. These sequences have homology to the nucleotide sequences shown in SEQ ID NO: 113-123 or SEQ ID NO: 124-134, and can express amino acids similar to SEQ ID NO: 91-101 and SEQ ID NO: 102-112, thereby can specifically bind to the IL-11 antigen to achieve the targeting function of the antibody.

In some preferred embodiments, the isolated nucleic acid molecule has a heavy chain nucleotide sequence shown in SEQ ID NO: 113-123 and a light chain nucleotide sequence shown in SEQ ID NO: 124-134. These nucleotide sequences are species optimized for easier expression in mammalian cells.

The present invention also provides an expression vector, which comprises the above-described isolated nucleic acid molecule. When the above-described isolated polynucleotide is ligated to a vector, the polynucleotide can be directly or indirectly connected to control elements on the vector, as long as these control elements can control the translation and expression and the like of the polynucleotide. Of course, these control elements can come directly from the vector itself, or can be exogenous, that is, not from the vector itself. Of course, it is sufficient that the polynucleotide is operably linked to the control elements. Herein, "operably linked" refers to linking an exogenous gene to a vector, so that the control elements in the vector, such as transcriptional control sequences and translational control sequences, etc., can perform the intended functions of regulating the transcription and translation of the exogenous gene. Of course, the polynucleotides used to encode the heavy chain and the light chain of the antibody can be independently inserted into different vectors, and usually inserted into the same vector. Commonly used vectors can be, for example, plasmid, phage, and the like. For example, Plasmid-X plasmid.

The present invention also provides a recombinant cell, which comprises the expression vector. The expression vector can be introduced into mammalian cells to construct and obtain recombinant cells, and then these recombinant cells can be used to express the antibody or antigen-binding fragment provided by the present invention. By culturing the recombinant cells, corresponding antibodies can be obtained. These usable mammalian cells can be, for example, CHO cells and the like.

### Pharmaceutical composition, kit and pharmaceutical use and use in the preparation of kit.

The present invention also provides a pharmaceutical composition, which comprises the above-mentioned antibody or antigen-binding fragment and a pharmaceutically acceptable carrier.

The anti-IL-11 antibody provided herein can be incorporated into pharmaceutical compositions suitable for administration to a subject. Typically, these pharmaceutical compositions include an anti-IL-11 antibody provided herein and a pharmaceutically acceptable carrier. "Pharmaceutically acceptable carrier" can include any and all solvents, dispersion media, coatings, antibacterial and antifungal agents, isotonic and absorption delaying agents, and the like that are physiologically compatible. Specific examples may be one or more of water, saline, phosphate buffered saline, dextrose, glycerol, ethanol, etc., and combinations thereof. In many cases, isotonic agents such as sugars, polyols (e.g., mannitol, sorbitol) or sodium chloride, etc. are included in the pharmaceutical compositions. Of course, the pharmaceutically acceptable carrier may also include minor amounts of auxiliary substances, such as wetting or emulsifying agents, preservatives or buffering agents, to prolong the shelf life or potency of the antibody.

For example, the antibody of the invention can be incorporated into pharmaceutical compositions suitable for parenteral administration (e.g., intravenous, subcutaneous, intraperitoneal, intramuscular). These pharmaceutical compositions can be prepared in various forms. For example, liquid, semisolid, and solid dosage form, and the like, include, but are not limited to, liquid solution (e.g., injection solution and infusion solution), dispersion or suspension, tablet, pill, powder, liposome and suppositorie. Typical pharmaceutical compositions are in the form of injection solution or infusion solution. The antibody can be administered by intravenous infusion or injection or intramuscular or subcutaneous injection.

Of course, the anti-IL-11 antibody herein can also be made into a kit or part of other diagnotic reagents as needed. According to the embodiments of the present invention, the present invention also provides a kit, which comprises the above-mentioned IL-11 antibody. The application of the kit provided by the present invention, for example, the kit can be used for immunoblotting, immunoprecipitation, etc., involving the use of the specific binding properties of IL-11 antigen and antibody for detection, and the like. These kits may contain any one or more of the following: antagonist, anti-IL-11 antibody, or drug reference material; protein purification column; immunoglobulin affinity purification buffer; assay diluent for cells; instruction or literature, etc. Anti-IL-11 antibody can be used in different types of diagnotic tests, such as in vitro or in vivo detection of various diseases or the presence of drugs, toxins, or other proteins. For example, it can be used to test for related diseases by testing the serum or blood of the subject. Such related diseases may include IL-11 related diseases, such as pulmonary fibrosis and the like. Of course, the antibody provided herein can also be used for radioimmunoassay and radioimmunotherapy of the above-mentioned diseases.

When using the anti-IL-11 antibody provided by the present invention to treat the above-mentioned diseases, the anti-IL-11 antibody provided by the present invention may be provided to the subject. To this end, the present invention provides a method for treating the above-mentioned diseases, comprising administering the antibody or antigen-binding fragment thereof provided by the present invention to a subject in need.

The scheme of the present invention will be explained below in conjunction with the embodiments. Those skilled in the art will understand that the following examples are only used to illustrate the present invention, and should not be construed as limiting the scope of the present invention. If no specific technique or condition is indicated in the examples, the technique or condition described in the literature in the field or the product instruction is used. The reagents or instruments used without the manufacturer's indication are conventional products that can be obtained from the market.

### Example 1 Production of recombinant human protein antigen hu-IL-11-His tag

Construction of vector: The amino acid sequence of human IL-11 was obtained from the global public database UniProt (P20809: 124294). An expression vector containing human IL-11 gene sequence was constructed using the method of molecular cloning. The vector encoding human IL-11 was transfected into mammalian cells CHO through electrotransfection for expression for 14 days. After fermentation, the cell supernatant was harvested, and Protein A purification column (GE healthcare, Cat: 17040201) was used for preliminary purification, then Superdex75 (GE healthcare, Cat: 29148721) was used for refine purification, finally the recombinant protein antigen hu-IL-11-His tag that can be used to immunize animals was obtained.

### Example 2 Animal immunization

In order to obtain the mice that can secret antibody against hu-IL-11, we immunized female Balb/c mice. The female Balb/c mice were purchased from Hunan SJA Laboratory Animal Co., Ltd. The recombinant protein antigen hu-IL-11-His tag and immune adjuvant were fully emulsified before animal immunization. The immunization process is shown in Table 1.

**Table 1: Schedule of Animal immunization**

| Immunization time point (Day) | Immunogen dose | Immune mode |
|---|---|---|
| 0 | 50ug hu-IL-11-His tag and CFA mixed in equal volume | Intraperitoneal multipoint injection |
| 14 | 50ug hu-IL-11-His tag and IFA mixed in equal volume | Intraperitoneal multipoint injection |
| 28 | 50ug hu-IL-11-His tag and IFA mixed in equal volume | Intraperitoneal multipoint injection |
| 35 | None | Orbital blood collection |
| 38 | 50ug hu-II,-11-His tag | Tail vein injection |
| 41 | None | The mice were sacrificed and the spleens were removed |

On the 35th day, a small amount of mice orbital blood was harvested, and the antibody titer was detected by the conventional Elisa method.

### Example 3 Fusion and screening of antibody-secreting cells

Isolated mouse spleen cells were fused with immortalized mouse myeloma cells SP2/0, then stood and cultured in RPMI-1640 complete medium containing 10⁻⁴ M Hypoxanthine, 4×10⁻⁷ M Aminopterin, 1.6×10⁻⁵ M Thymidine for about 14 days, the antibody affinity detection and screening were carried out after the fusion cells grew clone group. The positive clones that bound to IL-11 were selected, and the supernatants were taken, then flow cytometry was performed to determine the competitive binding of antigen and antibody. Cell lines with high secretion of specific antibody were selected for expanded culture and cryopreservation. The results are shown in Table 2. The specific method was to obtain an antibody strain with high affinity (OD450nm was the measured affinity value) for IL-11 through Elisa screening firstly, wherein P.C. was the binding affinity between the purchased IL-11 antibody (Abcam, ab130083) and IL-11 protein as a positive control. Secondly, by constructing a cell line stably expressing IL-11R, an appropriate amount of IL-11 protein was mixed with the positive hybridoma supernatant, then co-incubated with a cell line stably expressing IL-11R, the blocking effect (mean value of FITC-A) of hybridoma supernatant on IL-11/IL-11R was finally detected by flow cytometry, wherein N.C. was the value detected without hybridoma supernatant, which could be regarded as the value without blocking at all (Negative control). The underline in Table 2 indicates the selected clones. After multiple rounds of subcloning, a monoclonal antibody derived from a single antibody-secreting cell was finally obtained. The binding affinity (OD450nm) and the competitive blocking results of the monoclonal antibodies are shown in Table 3.

**Table 2: Test results of Elisa affinity test and flow cytometry competition test**

| Clone(No.) | OD(450nm) value | FITC-A mean | Clone(No.) | OD(450nm) value | FITC-A mean | Clone(No.) | OD(450nm) value | FITC-A mean |
|---|---|---|---|---|---|---|---|---|
| **3-1A2** | 2.243 | 12538 | **3-12F10** | 0.628 | 17067 | **5-5F5** | 2.768 | 4564 |
| **3-2A12** | 1.781 | 13334 | **3-13F11** | 1.915 | 15967 | **5-5F8** | 2.532 | 9436 |
| **3-2B2** | 1.084 | 13285 | **3-13G12** | 2.732 | 15312 | **5-5F11** | 2.627 | 12273 |
| **3-2B5** | 3.432 | 13837 | **3-13H1** | 1.41 | 13551 | **5-6F1** | 2.677 | 11496 |
| **3-3B9** | 1.777 | 13049 | **3-13H8** | 2.066 | 14323 | **5-6G5** | 2.679 | 10201 |
| **3-3G9** | 3.078 | 11577 | **3-15A11** | 1.214 | 16661 | **5-7B4** | 2.751 | 6587 |
| **3-3G11** | 2.943 | 13959 | **3-15C4** | 1.318 | 15017 | **5-9E12** | 2.049 | 10707 |
| **3-4G12** | 2.991 | 14418 | **3-15D5** | 2.894 | 11919 | **5-9D7** | 2.129 | 10127 |
| **3-4H1** | 2.876 | 12658 | **3-15D10** | 2.402 | 15064 | **5-9G8** | 3.591 | 6747 |
| **3-4H4** | 2.736 | 14459 | **3-17D12** | 2.48 | 15572 | **5-8G1** | 3.714 | 10460 |
| **3-4H12** | 3.33 | 13876 | **3-17E7** | 2.372 | 13301 | **5-10D3** | 3.201 | 10041 |
| **3-5B2** | 3.264 | 11001 | **3-18C11** | 2.566 | 14884 | **5-10F6** | 2.311 | 10472 |
| **3-5D11** | 1.068 | 13000 | **3-18C12** | 3.504 | 16114 | **5-12B1** | 2.351 | 9782 |
| **3-5G12** | 1.907 | 14267 | **3-19E8** | 3.412 | 15279 | **5-14B10** | 2.54 | 11385 |
| **3-6A4** | 2.656 | 750 | **3-20F12** | 3.496 | 14504 | **5-14C3** | 3.654 | 7672 |
| **3-7C1** | 2.661 | 13349 | **5-1A2** | 2.765 | 6217 | **5-17C11** | 3.462 | 11718 |
| **3-7C6** | 2.589 | 11605 | **5-1B12** | 2.865 | 4613 | **5-20E11** | 3.649 | 4330 |
| **3-7C9** | 2.722 | 11406 | **5-1E9** | 2.531 | 4638 | **5-21G2** | 3.545 | 4946 |
| **3-7G12** | 2.959 | 12412 | **5-2E11** | 2.921 | 5218 | **5-21H7** | 3.25 | 11385 |
| **3-7H5** | 1.995 | 13161 | **5-2F11** | 2.275 | 5625 | **5-22C4** | 3.561 | 6019 |
| **3-8H12** | 2.926 | 13360 | **5-2G11** | 2.948 | 5045 | **5-24F6** | 3.571 | 9954 |
| **3-9C12** | 1.915 | 14499 | **5-3E8** | 2.775 | 4897 | **5-25D3** | 1.87 | 3713 |
| **3-9D12** | 2.732 | 13833 | **5-3G12** | 2.77 | 8573 | **P.C.(Elisa)** | 3.62 | N/A |
| **3-9E1** | 2.894 | 13822 | **5-4C9** | 2.034 | 5933 | **N.C. (Flow)** | N/A | 24596 |

| | | | | | | | | |
|---|---|---|---|---|---|---|---|---|
| Note: N/A in Table 2 means: no data | | | | | | | | |

**Table 3: Test results of Elisa affinity test and flow cytometry competition test**

| Sample number | OD450 | FITC Mean | Sample number | OD450 | FITC Mean |
|---|---|---|---|---|---|
| 3-6A4 | 1.584 | 3036 | 3-5B2-2G4 | 1.65 | 19751 |
| 5-20E11-5F2 | 1.596 | 3109 | 5-21G12-1D3 | 1.528 | 3362 |
| 5-20E11-1A2 | 1.634 | 3635 | 3-5B2-7A4 | 1.636 | 20958 |
| 3-15D5-3A6 | 1.688 | 10944 | 3-9E1-3A2 | 1.544 | 9067 |
| 3-7C9-1A6 | 1.601 | 3148 | 3-15D5-6A1 | 1.511 | 13639 |
| 3-2B5-3D2 | 1.614 | 15289 | N.C.(flow) | N/A | 18433 |

| | | | | | |
|---|---|---|---|---|---|
| Note: N/A in Table 3 means: no data; because the sequence of 3-6A4 cells after subcloning was consistent with the sequence before subcloning, its number is the same as that in Table 2. | | | | | |

### Example 4 Sequence Determination of Monoclonal Antibodies

1^{∗}10^6~5^{∗}10^6 hybridoma cells were taken, the total RNA was extracted with Takara MiniBEST Universal RNA Extraction Kit, and cDNA was reverse transcribed with PrimeScript RT Reagent Kit (Takara Corporation). The above steps were carried out according to the instructions provided by the manufacturer. The design of gene amplification primers for antibody heavy chain variable region (VH) and light chain variable region (VL) mainly refers to the primer pairs in Ig-Primer Sets (Novagen Corporation, Cat. No. 69831-3), synthesized by BGI Genomics. The genes of heavy chain variable region and light chain variable region were amplified from the first strand of the cDNA, then cloned into the pMD18-T vector and transformed into E. coli DH5α, the clones were picked and sequenced by BGI Genomics to obtain the gene sequences of the heavy chain variable region (VH) and light chain variable region (VL). The primer sequences used and the sequenced antibody sequences are also found in the documentation.

### Example 5 Identification of antibody isotypes

The monoclonal hybridoma cell lines corresponding to the preferred antibody strains were injected into Balb/C mice by conventional method to produce and purify ascites antibodies. The purified antibody was used for antibody isotype analysis, and the antibody isotype detection method was detected by a mature kit (Proteintech, KMIM-2). The results are shown in Table 4 below.

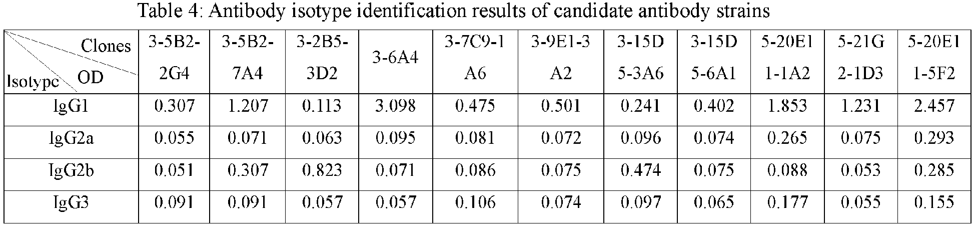

### Example 6 Expression and purification of preferred monoclonal antibodies

The sequences of preferred monoclonal antibodies 3-6A4 and 5-20E11-5F2 were constructed into conventional expression vectors by genetic engineering method, and then transfected into CHO cells. After fermentation, the collected cell culture supernatants were purified by a Protein G column (EzFast Protein G 4FF, Borgron), the balance buffer was 20mM PBS, 0.15M NaCl, pH7.4, and 0.1M glycine, pH3 .0±0.2 was used as the elution buffer. The protein eluate under the target absorption peak was collected, and after ultrafiltration with PBS buffer, part of the samples were taken for SDS-PAGE electrophoresis detection, and the aggregation content was determined by SEC-HPLC method. The aggregation contents of the antibodies were all within 3%.

### Example 7 Preferred antibodies competitively block the binding of IL-11 to IL-11R

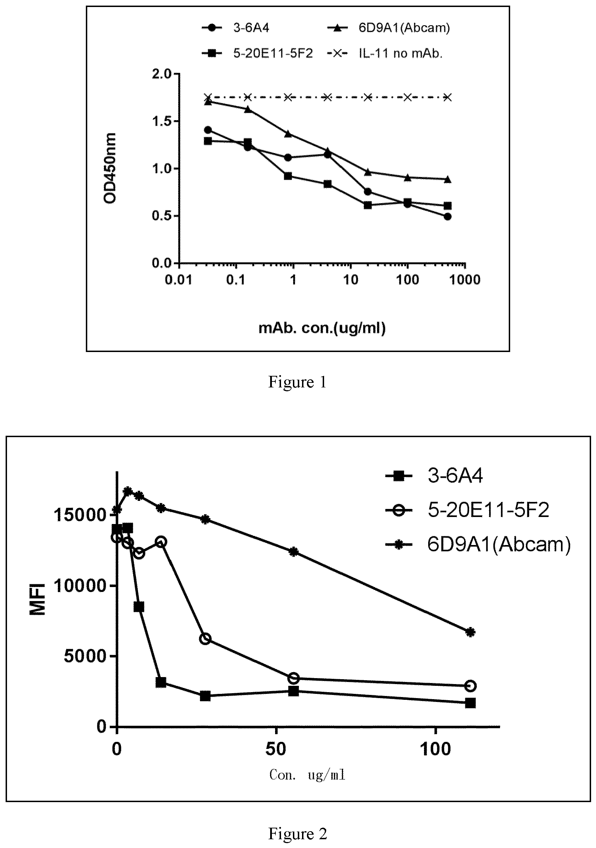

In order to detect the competitive binding ability of the preferred antibodies 3-6A4 and 5-20E11-5F2, the Elisa plates were coated with an appropriate amount of II,-11R-His protein and incubated overnight. Then, an appropriate amount of IL-11-Fc protein was added, and co-incubated with different concentration gradients of antibodies. Finally, HRP-conjugated Goat anti IgG-Fc secondary antibody (ThermoFisher, 31413) and TMB substrate were used for detection. We detected that both the preferred antibodies and 6D9A1 (Abcam, ab130083) could inhibit the binding of IL-11 to IL-11R to varying degrees. The results are shown in Figure 1.

### Example 8 Cross-reactivity of preferred antibodies

IL-6 cytokine family members include IL-6, IL-11, CNTF, CLC, LIF, CT-1, OSM, IL-27, IL-31. IL-11 is a member of the IL-6 cytokine family. We used conventional Elisa method to coat recombinant IL-6 (Sinobiological-10395-HNAE), IL-27 (R&D systems, 2526-IL-010), LIF (Peprotech, 300-05), CLC (R&D systems, 962-CL-050), IL-11 (LSBio, LS-G132887-10), OSM (Genscript-z03132), IL-31 (Peprotech, 200-31), CNTF (R&D systems, 257-NT-010), CT-1 (R&D systems, 612-CD-010) protein, and the binding affinity of preferred antibodies 3-6A4 and 5-20E11-5F2 to members of the IL-6 cytokine family was detected. The results show that the preferred antibodies do not cross-react with other members of the IL-6 cytokine family except for binding to the IL-11 cytokine. The Elisa results (OD450 nm) are shown in Table 5 below.

**Table 5: Binding affinity detection of preferred antibodies 3-6A4 and 5-20E11-5F2 to different members of the IL-6 cytokine family**

| | IL-6 | IL-27 | OSM | CLC | LIF | IL-31 | CNTF | CT-1 | IL-11 |
|---|---|---|---|---|---|---|---|---|---|
| 3-6A4 | 0.039 | 0.035 | 0.102 | 0.042 | 0.084 | 0.088 | 0.064 | 0.041 | 1.884 |
| 5-20E11-5F2 | 0.049 | 0.048 | 0.045 | 0.033 | 0.037 | 0.036 | 0.036 | 0.034 | 1.926 |

### Example 9 Binding affinity determination of preferred IL-11 monoclonal antibodies to antigen

Bio-Layer Interferometry (BLI) was used for binding affinity determination. Ni-NTA sensor was used to capture antigen IL-11 (His tag), the preferred antibodies 3-6A4 and 5-20E11-5F2 were used as analytes. The analytes were diluted in 7 concentration gradients, the binding time was 60s and the dissociation time was 1000s. The buffer was 20mM PBS, 0.15M NaCl, 0.1% BSA, 0.05% Tween 20, pH7. BLI kinetics/affinity software was used to analysis and obtain the dissociation constant (kdis) and affinity constant (KD) of the preferred antibodies and the corresponding hu-IL-11 ligand. The specific results are shown in Table 6 below.

**Table 6:**

| Sample ID | Cone. (nM) | Response | KD (M) | kon(1/Ms) | kon Error | kdis(1/s) | kdis Error | Full X^2 | Full R^2 |
|---|---|---|---|---|---|---|---|---|---|
| 3-6A4 | 41.7 | 1.0442 | 3.93E-11 | 1.06E+06 | 2.08E+04 | 4.16E-05 | 2.39E-06 | 0.017992 | 0.999488 |
| 5-20E11-5F2 | 166.7 | 1.2727 | 6.90E-11 | 1.75E+05 | 3.19E+03 | 1.21E-05 | 2.94E-06 | 0.051363 | 0.999171 |

### Example 10 Competitive blocking activity detection of preferred antibodies

In order to evaluate the binding of the preferred Anti-huIL-11 antibodies 3-6A4 and 5-20E11-5F2 to IL-11 and whether the binding between the ligand and the receptor can be blocked, we constructed a cell line (membrane-II,-11R CHO) that stably expressing IL-11R. The blocking activities of candidate antibodies and commercial antibody 6D9A1 (Abcam, ab130083) against IL-11 and IL-11R were detected by flow cytometry. Firstly, an appropriate amount of IL-11 recombinant protein with Fc tag was co-incubated with different gradients of antibodies for 15 mins, and then incubated with 1 E+05 membrane-II,-11R CHO cells. The secondary antibody used in the detection was a secondary antibody conjugated with a FITC fluorophore (Goat-anti-Fc-FITC) (Abcam, ab98529). The competitive binding results show that the neutralizing activity IC50 of the 3-6A4 antibody is about 7µg/ml; the neutralizing activity IC50 of the 5-20E11-5F2 antibody is about 21µg/ml, and the blocking activity of the candidate antibodies is significantly better than that of the 6D9A1 clone antibody, the results are shown in Figure 2.

### Example 11 Preferred antibodies inhibit the transformation of lung fibroblasts HFL-1 into fibrosis

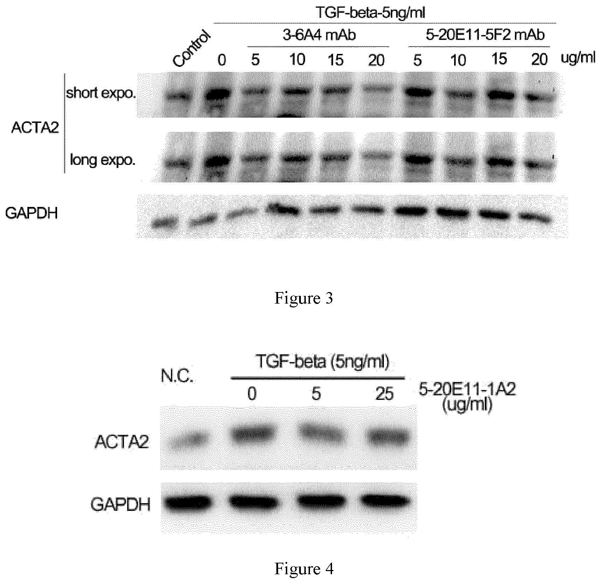

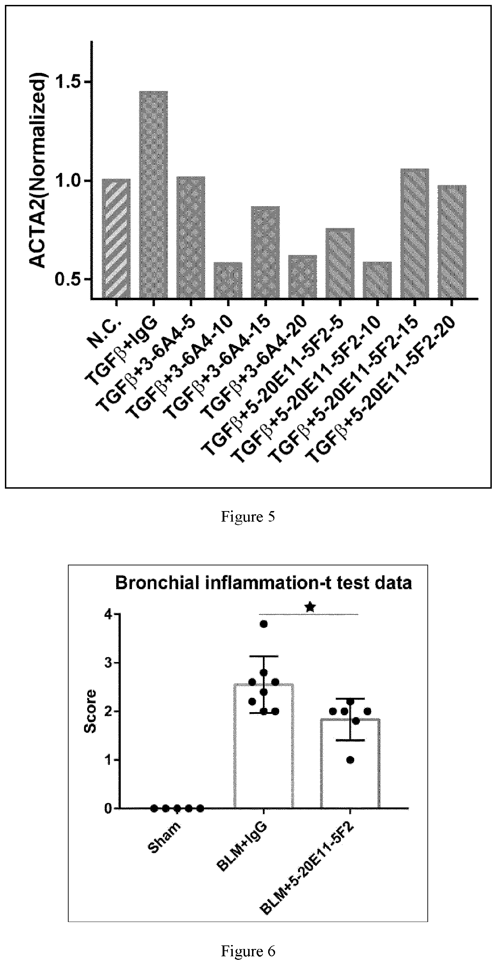

As lung fibroblasts, HFL-1 cells were induced by pro-fibrotic factors such as TGF-beta1 and expressed a large amount of IL-11 cytokines, and finally transformed into fibrosis. When HFL-1 cells were transformed into fibrosis, the marker ACTA2 protein was accumulated continuously. In this example, when TGF-beta1 cytokine was used to induce HFL-1 cells to transform into fibrosis, the preferred antibodies 3-6-A4, 5-20E11-1A2 and 5-20E11-5F2 were added at the same time. After culturing for 48h, the cells were harvested for western blot detection analysis. It can be seen that different concentrations of antibodies can inhibit the transformation of HFL-1 cells to fibrosis (the expression of ACTA2 protein was reduced), as shown in Figure 3, Figure 4 and the corresponding statistical analysis Figure 5.

### Example 12 Preferred antibodies inhibit pulmonary fibrosis in C57BL/6 mice

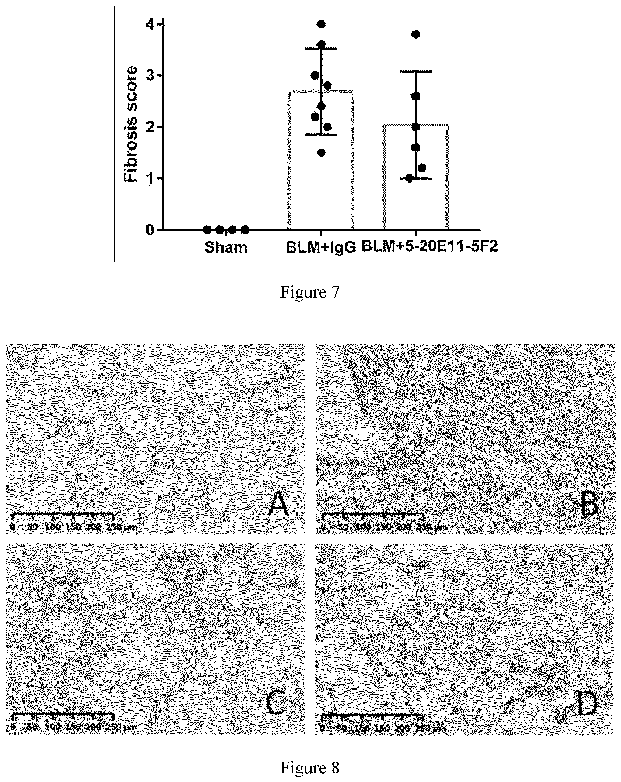

In an animal model of pulmonary fibrosis in C57BL/6 mice induced by bleomycin, specifically, 10-week-old C57BL/6 was subjected to general anesthesia with pentobarbital anesthetic and the main trachea of the mouse lung was surgically exposed. Then an appropriate amount of bleomycin was injected into the trachea by instillation or nebulization. After seven days of bleomycin induction in mice, pulmonary fibrosis was basically appeared. On the eighth day, the preferred antibody 5-20E11-5F2 or the isotype control antibody IgG was injected every other day. After 14 days of continuous injection, the mice were sacrificed and the lungs were removed, and the inflammatory damage and fibrosis scores of the lungs were detected. Under this pharmacodynamic model, it can be seen that the candidate antibodies can slow down pulmonary fibrosis in mice, and the results are shown in Figures 6 and 7.

### Example 13 Preferred antibodies inhibit pulmonary fibrosis in Sprague Dawley rats

Intratracheal instillation of bleomycin in rats was a widely used pharmacodynamic model to study pulmonary fibrosis, the ability of the preferred antibodies to reduce bleomycin-induced unilateral pulmonary fibrosis in rats in vivo was tested in this experiment.

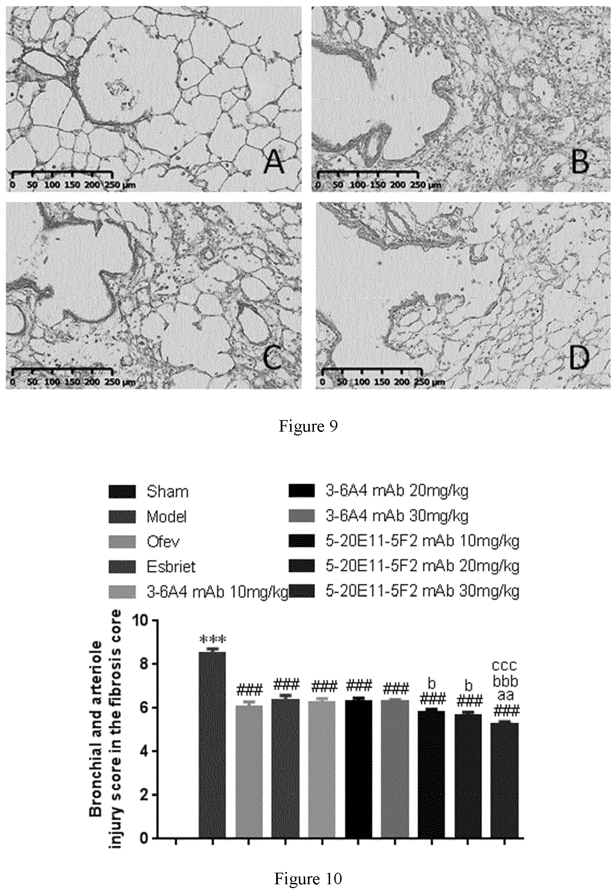

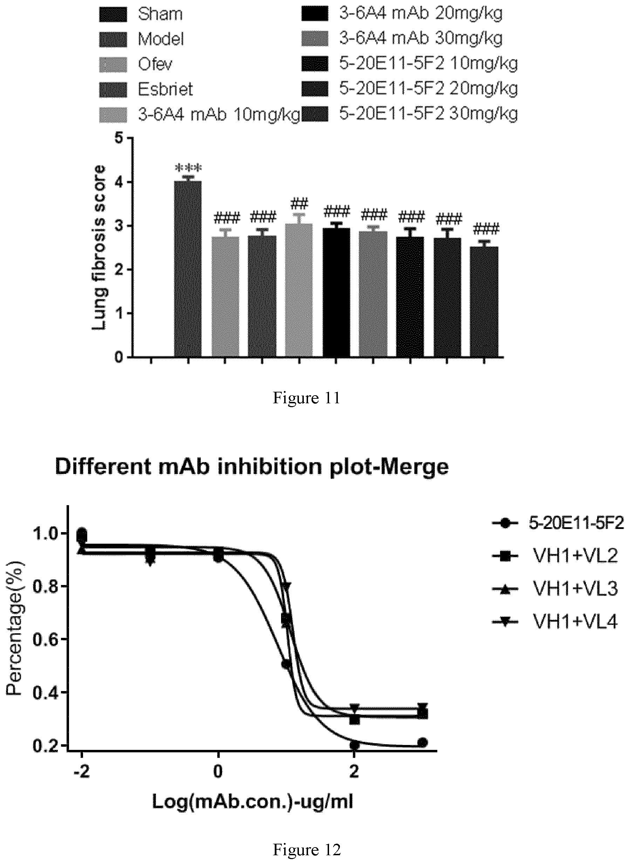

The male animals were divided into nine groups. The depth of anesthesia was maintained by 25 mg/kg pentobarbital sodium combined with 1-2.5% isoflurane respiratory anesthesia. The fibrosis groups were induced by tracheal injection of bleomycin, and in sham-operated group, the bleomycin was replaced by an equal volume of saline. On the eighth day of model establishment, different groups were treated with different doses of the preferred antibody for 14 days every other day, wherein pirfenidone (Esbriet) or nintedanib (Ofev) were used as the treatment reference (Esbriet and Ofev were both used 100 mg/kg/day). After the treatment, lungs were taken for immunohistochemical H&E staining and Masson's tri-staining. The histological examination of the rat lungs showed that the control group was normal. In the bleomycin-treated lungs, the inflammatory damage was significantly aggravated, and the preferred antibody could significantly alleviate the infiltration of inflammatory cells in the lungs, as shown in Figure 8 (Figure 8, H&E staining of lung tissue, A is the control group; B is the bleomycin-induced group; C is the 3-6A4 mAb-20mg/kg group; D is the 5-20E11-5F2 mAb-20mg/kg group). In addition, the preferred antibodies could also greatly alleviate the process of pulmonary fibrosis, as shown in Figure 9 (Figure 9, Masson's tri-staining of lung tissue, A is the control group; B is the bleomycin-induced group; C is the 3-6A4 mAb-20mg/kg group; D is 5-20E11-5F2 mAb-20mg/kg). The statistical analysis results show that the preferred antibodies can alleviate the inflammatory damage and fibrosis process of rat lung caused by bleomycin at different doses, as shown in Figure 10 (Figure 10, ###P<0.001 vs. BLM Model; aaP<0.01 vs. Ofev; bP<0.05 vs. Esbriet, bbbP<0.001 vs. Esbriet; cccP<0.001 vs. 3-6A4 mAb 30mg/kg), Figure11 (***P<0.001 vs. Sham; ##P<0.01 vs. BLM Model, ###P<0.001 vs. BLM Model.).

### Example 14 Humanization of IL-11 antibody

The humanization of the IL-11 antibody in the above examples was carried out by complementarity determining region grafting (CDR grafting). Firstly, the human germline antibody sequence with the highest homology to the light and heavy chain variable regions of murine antibody was found. The germline selected for humanization of the heavy chain variable region of the antibody was IGHV1-46*01, IGKV3-11*01 was selected for humanization of the light chain variable region. The CDR region of the murine antibody was retained, and the framework sequence of the murine antibody was replaced with the framework sequence of the human germline antibody. Secondly, the structure model of the murine antibody Fv fragment was established, and BLAST was performed in the PDB antibody database, and the structure INQB was selected as the template for homology modeling. Each different amino acid position in the structural model of human antibody and the corresponding murine antibody was compared one by one, the amino acid that did not meet the FR structural specification, the amino acid located in the FR core, and the amino acid located at the VH-VL interface were selected and backmutated to murine sequences.

The sequences of the obtained heavy chain variable region and the light chain variable region of the IL-11 humanized antibody are shown in SEQ ID NO: 143-146 and SEQ ID NO: 147-150.
Sequence of IL-11 humanized antibody heavy chain variable region (VH1): 
QVQLVQSGAEVKKPGASVKVSCKASDYTFTNYWLGWVRQAPGQGLEWMGHIYP
GGGYTNYNEKFKGRVTMTRDTSTSTVYMELSSLRSEDTAVYYCARVRSGNDALDFWGQ
GTLVTVSS (SEQ ID NO: 143).
Sequence of IL-11 humanized antibody heavy chain variable region (VH2): 
QVQLVQSGAEVKKPGASVKVSCKASDYTFTNYWLGWVRQAPGQGLEWIGHIYPG
GGYTNYNEKFKGRVTMTADTSTSTVYMELSSLRSEDTAVYFCARVRSGNDALDFWGQG
TLVTVSS (SEQ ID NO: 144).
Sequence of IL-11 humanized antibody heavy chain variable region (VH3): 
QVQLVQSGAEVKKPGASVKVSCKASDYTFTNYWLGWVRQAPGQGLEWIGHIYPG
GGYTNYNEKFKGRATLTADTSTSTVYMELSSLRSEDTAVYFCARVRSGNDALDFWGQG
TLVTVSS (SEQ ID NO: 145).
Sequence of IL-11 humanized antibody heavy chain variable region (VH4): 
QVQLVQSGAEVKKPGASVKVSCKASDYTFTNYWLGWVRQAPGHGLEWIGHIYPG
GGYTNYNEKFKGRATLTADTSTSTVYMELSSLRSEDTAVYFCARVRSGNDALDFWGQG
TLVTVSS (SEQ ID NO: 146).
Sequence of IL-11 humanized antibody light chain variable region (VL1): 
EIVLTQSPATLSLSPGERATLSCRASESVDEYGISFMNWYQQKPGQAPRLLIYSASNQ
GSGIPARFSGSGSGTDFTLTISSLEPEDFAVYYCQQSKEVPWTFGGGTKLEIK (SEQ ID NO:
147).
Sequence of IL-11 humanized antibody light chain variable region (VL2): 
EIVLTQSPATLSLSPGERATLSCRASESVDEYGISFMNWYQQKPGQAPRLLIYSASNQ
GSGIPARFSGSGSGTDFTLTISSLEPEDFAVYFCQQSKEVPWTFGGGTKLEIK (SEQ ID NO:
48).
Sequence of IL-11 humanized antibody light chain variable region (VL3): 
EIVLTQSPATLSLSPGERATLSCRASESVDEYGISFMNWFQQKPGQAPRLLIYSASNQ
GSGIPARFSGSGSGTDFTLTISSLEPEDFAVYFCQQSKEVPWTFGGGTKLEIK (SEQ ID NO:
149).
Sequence of IL-11 humanized antibody light chain variable region (VL4):

EIVLTQSPATLSLSPGERATLSCRASESVDEYGISFMNWFQQKPGQAPRLLIYSASNQ
GSGIPARFSGSGSGTDFTLTISSLEPEDFAMYFCQQSKEVPWTFGGGTKLEIK (SEQ ID

NO: 150).

The nucleic acid sequences encoding the light chain and heavy chain of the IL-11 humanized antibody were synthesized and inserted into the eukaryotic expression vector pcDNA3.4. 4 heavy chain sequence vectors were paired respectively with 4 light chain sequence vectors and co-transfected into mammalian cells CHO for expression to obtain a single-chain antibody in the form of scFv. One week later, the supernatant containing the scFv antibodies were collected for affinity detection.

### Example 15 Affinity ranking of IL-11 humanized antibody

Biacore was used to measure the binding affinity of each humanized antibody supernatant scFv to IL-11, and sorted according to the dissociation constant kd(1/s), and three humanized antibodies with the smallest dissociation constant kd(1/s) were selected. The measure method included the steps of capturing the humanized antibody at a flow rate of 10 ul/min. The flow rate was switched to 30ul/min, 400nM of IL-11 antigen was flowed through the sample channel, the binding time was 180s, and the dissociation time was 600s. Finally the chip was regenerated with 10 mM glycine buffer. The results are shown in Table 7. The humanized antibodies VH1+VL2, VH1+VL3, VH1+VL4 dissociated from IL-11 at a slower rate than other humanized antibodies.

**Table 7: Affinity of IL-11 Humanized Antibodies to IL-11 Antigen**

| Ligand | Analyte | ka (1/Ms) | kd (1/s) | KD (M) | Rmax (RU) | Chi² (RU²) |
|---|---|---|---|---|---|---|
| BLANK(PBS) | IL-11 | NA | NA | NA | NA | NA |
| 5-20E11-5F2 | IL-11 | 5.84E+04 | 1.22E-04 | 2.09E-09 | 213.2 | 1.14E-01 |
| NC(culture medium) | IL-11 | NA | NA | NA | NA | NA |
| VH1+VL1-scFv | IL-11 | 3.51E+04 | 7.58E-04 | 2.16E-08 | 257.1 | 8.24E-01 |
| VH1+VL2-scFv | IL-11 | 4.13E+04 | 4.61E-04 | 1.12E-08 | 247.2 | 5.66E-01 |
| VH1+VL3-scFv | IL-11 | 3.55E+04 | 4.36E-04 | 1.23E-08 | 239.7 | 4.35E-01 |
| VH1+VL4-scFv | IL-11 | 3.31E+04 | 3.87E-04 | 1.17E-08 | 268.7 | 3.92E-01 |
| VH2+VL1-scFv | IL-11 | 5.34E+04 | 8.90E-04 | 1.67E-08 | 142.3 | 7.35E-01 |
| VH2+VL2-scFv | IL-11 | 4.57E+04 | 8.12E-04 | 1.78E-08 | 208.9 | 7.67E-01 |
| VH2+VL3-scFv | IL-11 | 4.56E+04 | 5.41E-04 | 1.19E-08 | 185.7 | 4.06E-01 |
| VH2+VL4-scFv | IL-11 | 4.88E+04 | 5.28E-04 | 1.08E-08 | 174.5 | 4.64E-01 |
| VH3+VL1-scFv | IL-11 | 4.45E+04 | 1.00E-03 | 2.25E-08 | 184.8 | 8.66E-01 |
| VH3+VL2-scFv | IL-11 | 4.65E+04 | 6.80E-04 | 1.46E-08 | 184.4 | 4.71E-01 |
| VH3+VL3-scFv | IL-11 | 4.76E+04 | 4.64E-04 | 9.76E-09 | 181.4 | 4.14E-01 |
| VH3+VL4-scFv | IL-11 | 4.18E+04 | 5.56E-04 | 1.33E-08 | 219.5 | 5.76E-01 |
| VH4+VL1-scFv | IL-11 | 4.89E+04 | 8.40E-04 | 1.72E-08 | 128.4 | 4.84E-01 |
| VH4+VL2-scFv | IL-11 | 5.52E+04 | 6.14E-04 | 1.11E-08 | 135.9 | 4.22E-01 |
| VH4+VL3-scFv | IL-11 | 4.61E+04 | 5.16E-04 | 1.12E-08 | 181.7 | 3.90E-01 |
| VH4+VL4-scFv | IL-11 | 4.79E+04 | 4.81E-04 | 1.00E-08 | 147.6 | 2.54E-01 |

### Example 16 Expression and purification of preferred IL-11 humanized antibodies

The preferred IL-11 humanized antibodies VH1+VL2, VH1+VL3, VH1+VL4 were respectively constructed with full-length sequences (including Fc part), and transfected into mammalian cells CHO for expression. After fermentation, the collected cell culture supernatants were purified by Protein A column, the balance buffer was 20mM PBS, pH7.4, and the eluent was 0.1M glycine pH3.0±0.2, and the protein eluent under the target absorption peak was collected. After ultrafiltration with PBS buffer, part of the samples were taken to measure the concentration by HPLC and purity by SDS-PAGE electrophoresis detection.

### Example 17 Affinity detection of preferred IL-11 humanized antibodies

The binding affinity of each preferred humanized full-length (IgG format) antibody sample to IL-11 was determined by Biacore. The measure method included the steps of capturing the humanized antibody at a flow rate of 10 ul/min. The flow rate was switched to 30ul/min, and different concentrations of IL-11 antigen (400nM, 200nM, 100nM, 50nM, 25nM, 12.5nM, 6.25nM) were flowed through the sample channel and reference channel, the binding time was 180s, and the dissociation time was 600s. Finally the chip was regenerated with 10 mM glycine buffer. As shown in Table 8, the affinity of the humanized antibody VH1+VL3 was better than that of the other two humanized antibodies, and slightly lower than that of the 5-20E11-5F2 antibody.

**Table 8: Affinity of preferred humanized antibodies to IL-11 antigen**

| Ligand | Analyte | ka (1/Ms) | kd (1/s) | KD (M) | Rmax (RU) | Chi² (RU²) |
|---|---|---|---|---|---|---|
| VH1+VL2 | IL-11 | 4.98E+04 | 5.97E-04 | 1.20E-08 | 114.1 | 4.03E-01 |
| VH1+VL3 | IL-11 | 5.26E+04 | 4.74E-04 | 9.03E-09 | 105.6 | 3.71E-01 |
| VH1+VL4 | IL-11 | 5.43E+04 | 5.45E-04 | 1.00E-08 | 63.26 | 1.25E-01 |
| 5-20E11-5F2 | IL-11 | 6.76E+04 | 4.33E-04 | 6.41E-09 | 72.58 | 6.99E-02 |

### Example 18 Competitively blocking the binding of IL-11 to IL-11R with preferred humanized antibodies

To assess whether the preferred humanized antibodies could block the binding of IL-11 to IL-11R, a cell line (membrane-IL-11R CHO) that stably expressing IL-11R was constructed. The measure method included the following steps: IL-11-Fc protein with a concentration of 100 µg/ml was taken and added to a 96-well U-shaped plate with 10 ul/well, and incubated with 90ul of serial dilutions of preferred humanized antibodies (100ug/ml, 10ug/ml, 1ug/ml, 0.1ug/ml, 0.01ug/ml, 0.001ug/ml) at room temperature for 1h. Then the plate was incubated with 1.5^{∗}10^5 membrane-II,-11R CHO cells (100ul/well) for 1h at room temperature. After incubation, the plate was centrifuged at 1500rpm for 5 min, washed three times with PBS, and 1:400 diluted flow secondary antibody Goat-anti- Fc-FITC (Abcam, ab98529) was added with 200 ul/well. Then, the plate was incubated at 4°C for 1h, centrifuged at 1500rpm for 5min, washed three times with PBS, resuspended cells in 180ul PBS, and detected by flow cytometry. The results are plotted as an S-curve as shown in Figure 12. The results show that the blocking activity of the humanized antibody was basically the same as that of the 5-20E11-5F2 antibody.

**Table 9 EC50 of humanized antibody competitively blocking the binding of IL-11 to IL-11R**

| Antibody number | 5-20E11-5F2 | VH1+VL2 | VH1+VL3 | VH1+VL4 |
|---|---|---|---|---|
| EC50 (ug/ml) | 7.537 | 11.57 | 10.46 | 12.48 |

### Example 19 Preferred humanized antibodies inhibit the transformation of hepatic fibroblasts LX-2 to fibrosis

LX-2 cells are human hepatic stellate cells, which can express a large amount of IL-11 cytokines when induced by pro-fibrotic factors such as TGF-beta1, and finally transform into fibrosis. When LX-2 cells transform into fibrosis, the marker ACTA2 protein will continue to accumulate. In this example, LX-2 cells were plated in a 6-well plate at a density of 3^{∗}10^5 cells/well and cultured for 10h until the cells adhered. After starvation in serum-free DMEM medium for 12h, 5 ng/ml of TGF-beta1 cytokine was added for stimulation, and serial dilutions of preferred humanized antibodies (50ug/ml, 25ug/ml, 5ug/ml) were added at the same time. After culturing for 24h, the medium was taken, 200ul of RIPA lysis solution (Biyuntian, P0013B) was added to each well for western blot analysis. The results show that different concentrations of humanized antibodies can inhibit the transformation of LX-2 cells into fibrosis (the expression of ACTA2 protein was decreased).

In the description of this specification, description with reference to the term "an embodiment," "some embodiments," "example," "specific example," or "some examples", etc., means that a particular feature, structure, material, or characteristic described in connection with the embodiment or example is included in at least one embodiment or example of the present invention. In this specification, schematic representations of the above terms are not necessarily referring to the same embodiment or example of the present disclosure. Furthermore, the particular features, structures, materials, or characteristics may be combined in any suitable manner in one or more embodiments or examples. In addition, those skilled in the art can integrate and combine different embodiments, examples or the features of them as long as they are not contradictory to one another.

Although the embodiments of the present invention have been shown and described above, it would be appreciated that the above-mentioned embodiments are exemplary and should not be construed to limit the present disclosure, changes, alternatives, and modifications to the above-described embodiments may be made by those of ordinary skill in the art within the scope of the present invention.

## Claims

1. An antibody or an antigen-binding fragment thereof that can specifically recognize IL-11, wherein the antibody comprises CDRs selected from at least one of the following or an amino acid sequence having at least 95% identity thereto:
a heavy chain variable region comprising CDRs having the amino acid sequence of SEQ ID NO: 1-33,
a light chain variable region comprising CDRs having the amino acid sequence of SEQ ID NO: 34-66.

2. The antibody or antigen-binding fragment thereof of claim 1, wherein the antibody comprises:
a heavy chain variable region comprising CDR1, CDR2, CDR3 as shown in SEQ ID NO: 1, 2 and 3 or an amino acid sequence having at least 95% identity to SEQ ID NO: 1, 2 and 3, respectively; or
a heavy chain variable region comprising CDR1, CDR2, CDR3 as shown in SEQ ID NO:4, 5 and 6 or an amino acid sequence having at least 95% identity to SEQ ID NO:4, 5 and 6, respectively; or
a heavy chain variable region comprising CDR1, CDR2, CDR3 as shown in SEQ ID NO:7, 8 and 9 or an amino acid sequence having at least 95% identity to SEQ ID NO:7, 8 and 9, respectively; or
a heavy chain variable region comprising CDR1, CDR2, CDR3 as shown in SEQ ID NO: 10, 11 and 12 or an amino acid sequence having at least 95% identity to SEQ ID NO: 10, 11 and 12, respectively; or
a heavy chain variable region comprising CDR1, CDR2, CDR3 as shown in SEQ ID NO: 13, 14 and 15 or an amino acid sequence having at least 95% identity to SEQ ID NO: 13, 14 and 15, respectively; or
a heavy chain variable region comprising CDR1, CDR2, CDR3 as shown in SEQ ID NO: 16, 17 and 18 or an amino acid sequence having at least 95% identity to SEQ ID NO: 16, 17 and 18, respectively; or
a heavy chain variable region comprising CDR1, CDR2, CDR3 as shown in SEQ ID NO: 19, 20 and 21 or an amino acid sequence having at least 95% identity to SEQ ID NO: 19, 20 and 21, respectively; or
a heavy chain variable region comprising CDR1, CDR2, CDR3 as shown in SEQ ID NO:22, 23 and 24 or an amino acid sequence having at least 95% identity to SEQ ID NO:22, 23 and 24, respectively; or
a heavy chain variable region comprising CDR1, CDR2, CDR3 as shown in SEQ ID NO:25, 26 and 27 or an amino acid sequence having at least 95% identity to SEQ ID NO:25, 26 and 27, respectively; or
a heavy chain variable region comprising CDR1, CDR2, CDR3 as shown in SEQ ID NO:28, 29 and 30 or an amino acid sequence having at least 95% identity to SEQ ID NO:28, 29 and 30, respectively; or
a heavy chain variable region comprising CDR1, CDR2, CDR3 as shown in SEQ ID NO:31, 32 and 33 or an amino acid sequence having at least 95% identity to SEQ ID NO: 31, 32 and 33, respectively.

3. The antibody or antigen-binding fragment thereof of claim 1, wherein the antibody comprises:
a light chain variable region comprising CDR1, CDR2, CDR3 as shown in SEQ ID NO:34, 35 and 36 or an amino acid sequence having at least 95% identity to SEQ ID NO:34, 35 and 36, respectively; or
a light chain variable region comprising CDR1, CDR2, CDR3 as shown in SEQ ID NO:37, 38 and 39 or an amino acid sequence having at least 95% identity to SEQ ID NO:37, 38 and 39, respectively; or
a light chain variable region comprising CDR1, CDR2, CDR3 as shown in SEQ ID NO:40, 41 and 42 or an amino acid sequence having at least 95% identity to SEQ ID NO:40, 41 and 42, respectively; or
a light chain variable region comprising CDR1, CDR2, CDR3 as shown in SEQ ID NO:43, 44 and 45 or an amino acid sequence having at least 95% identity to SEQ ID NO:43, 44 and 45, respectively; or
a light chain variable region comprising CDR1, CDR2, CDR3 as shown in SEQ ID NO:46, 47 and 48 or an amino acid sequence having at least 95% identity to SEQ ID NO:46, 47 and 48, respectively; or
a light chain variable region comprising CDR1, CDR2, CDR3 as shown in SEQ ID NO:49, 50 and 51 or an amino acid sequence having at least 95% identity to SEQ ID NO:49, 50 and 51, respectively; or
a light chain variable region comprising CDR1, CDR2, CDR3 as shown in SEQ ID NO:52, 53 and 54 or an amino acid sequence having at least 95% identity to SEQ ID NO:52, 53 and 54, respectively; or
a light chain variable region comprising CDR1, CDR2, CDR3 as shown in SEQ ID NO:55, 56 and 57 or an amino acid sequence having at least 95% identity to SEQ ID NO:55, 56 and 57, respectively; or
a light chain variable region comprising CDR1, CDR2, CDR3 as shown in SEQ ID NO:58, 59 and 60 or an amino acid sequence having at least 95% identity to SEQ ID NO:58, 59 and 60, respectively; or
a light chain variable region comprising CDR1, CDR2, CDR3 as shown in SEQ ID NO:61, 62 and 63 or an amino acid sequence at least 95% identity to SEQ ID NO: 61, 62 and 63.
a light chain variable region comprising CDR1, CDR2, CDR3 as shown in SEQ ID NO:64, 65 and 66 or an amino acid sequence at least 95% identity to SEQ ID NO: 64, 65 and 66.

4. The antibody or antigen-binding fragment thereof of claim 1, wherein the antibody comprises at least one of a heavy chain framework region sequence and a light chain framework region sequence, and at least a part of at least one of the heavy chain framework region sequence and the light chain framework region sequence is derived from at least one of a murine antibody, a human antibody, a primate antibody, or a mutant thereof.

5. The antibody or antigen-binding fragment thereof of claim 1, wherein the heavy chain variable region has the amino acid sequence shown in any one of SEQ ID NO: 67-77.

6. The antibody or antigen-binding fragment thereof of claim 1, wherein the light chain variable region has the amino acid sequence shown in any one of SEQ ID NO: 78-88.

7. The antibody or antigen-binding fragment thereof of claim 1, wherein the antibody comprises at least one of a heavy chain constant region and a light chain constant region, and at least a part of at least one of the heavy chain constant region and the light chain constant region is derived from at least one of a murine antibody, a human antibody, a primate antibody, or a mutant thereof.

8. The antibody or antigen-binding fragment thereof of claim 1, wherein both the light chain constant region and the heavy chain constant region of the antibody are derived from a human IgG antibody or a mutant thereof.

9. The antibody or antigen-binding fragment thereof of claim 1, wherein the light chain constant region of the antibody is derived from the light chain constant region of human Kappa; the heavy chain constant region is derived from the heavy chain constant region of human IgG1.

10. The antibody or antigen-binding fragment thereof of claim 7, wherein the full-length sequence of the antibody constant region is shown in SEQ ID NO: 89 or 90.

11. The antibody or antigen-binding fragment thereof of claim 10, wherein the heavy chain has the amino acid sequence shown in any one of SEQ ID NO: 91-101 and the light chain has the amino acid sequence shown in any one of SEQ ID NO: 102-112.

12. The antibody or antigen-binding fragment thereof of claim 1, wherein the antibody is a single-chain antibody, a multimeric antibody, a CDR-grafted antibody or a small molecule antibody.

13. The antibody or antigen-binding fragment thereof of claim 12, wherein the single-chain antibody comprises a heavy chain variable region with the amino acid sequence shown in any one of SEQ ID NO: 67-77 and a light chain variable region with the amino acid sequence shown in any one of SEQ ID NO: 78-88, wherein the C-terminus of the heavy chain variable region is connected to the N-terminus of the light chain variable region through a connecting peptide linker, or the C-terminus of the light chain variable region is connected to the N-terminus of the heavy chain variable region through a connecting peptide linker.

14. The antibody or antigen-binding fragment thereof of claim 12, wherein the small molecule antibody comprises at least one of Fab antibody, Fv antibody, single domain antibody and minimum recognition unit.

15. A nucleic acid molecule encoding the antibody or antigen-binding fragment thereof of any one of claims 1-14.

16. The nucleic acid molecule of claim 15, wherein the nucleic acid molecule is DNA.

17. The nucleic acid molecule of claim 16, wherein the nucleic acid molecule has a nucleotide sequence shown in any one of SEQ ID NO: 113-123 or a nucleotide sequence shown in any one of SEQ ID NO: 124-134.

18. An expression vector carrying the nucleic acid molecule of any one of claims 15-17.

19. The expression vector of claim 18, wherein the expression vector is a eukaryotic expression vector.

20. A recombinant cell carrying the nucleic acid molecule of any one of claims 15-17, or expressing the antibody or antigen-binding fragment thereof of any one of claims 1-14.

21. The recombinant cell of claim 20, wherein the recombinant cell is obtained by introducing the expression vector of claim 18 or 19 into a host cell.

22. The recombinant cell of claim 21, wherein the expression vector is introduced into the host cell by electrotransduction.

23. The recombinant cell of claim 20, wherein the recombinant cell is a eukaryotic cell.

24. The recombinant cell of claim 20, wherein the recombinant cell is a mammalian cell.

25. A pharmaceutical composition comprising the antibody of any one of claims 1-14, the nucleic acid molecule of any one of claims 15-17, the expression vector of any one of claims 18-19 or the recombinant cell of any one of claims 20-24.

26. Use of the antibody of any one of claims 1-14, the nucleic acid molecule of any one of claims 15-17, the expression vector of any one of claims 18-19 or the recombinant cell of any one of claims 20-24, the pharmaceutical composition of claim 25 in the manufacture of a medicament for treating or preventing pulmonary fibrosis, non-alcoholic steatohepatitis, chronic heart failure or diseases caused by the transformation of fibroblasts into fibrosis.

27. A kit for detecting IL-11 comprising the antibody of any one of claims 1-14.

28. Use of the antibody of any one of claims 1-14, the nucleic acid molecule of any one of claims 15-17, the expression vector of any one of claims 18-19 or the recombinant cell of any one of claims 20-24 in the preparation of a kit, wherein the kit is used for detecting IL-11 or diagnosing IL-11-related diseases.
